# EUROPÄISCHE PATENTSCHRIFT

(11) **EP 0 795 640 B1**
(45) Veröffentlichungstag und Bekanntmachung des Hinweises auf die Patenterteilung: **18.06.2008**
(21) Anmeldenummer: 97810121.0
(22) Anmeldetag: 04.03.1997
(51) Int. Cl.: D06M 13/358, D06M 13/248, D06P 1/642, C07D 251/20

(54) **Stabilisatorkombination**
Combination of stabilizers
Combinaison de stabilisants

(30) Priorität: 13.03.1996 CH 66396
(43) Veröffentlichungstag der Anmeldung: 17.09.1997
(73) Patentinhaber: Huntsman Advanced Materials (Switzerland) GmbH, 4057 Basel (CH)
(72) Erfinder: Tittmann, Rolf, 79540 Lörrach (DE); Fuso, Francesco, 4106 Therwil (CH); Reinert, Gerhard, 4123 Allschwil (CH); Härri, Hans Peter, 4153 Reinach (CH)

(56) Entgegenhaltungen:
- EP-A- 0 584 044
- DE-A- 1 670 332
- DE-B- 1 241 452
- GB-A- 1 176 770
- HELV. CHIM. ACTA, Bd. 55, Nr. 1, 1972, BASEL, Seiten 1566-1595, XP002034121 BRUNETTI, H.; LÜTHI, C. E.: "Die Synthese von aromatisch substitutierten o-Hydroxyphenyl-s-triazinen"

## Beschreibung

Die vorliegende Erfindung betrifft neue Stabilisatorgemische enthaltend Mono- und Bis-Resorcinyltriazine, Verfahren zu ihrer Herstellung sowie ihre Verwendung zur photochemischen und chemischen Stabilisierung von organischem Material, vorzugsweise von ungefärbten oder gefärbten Textilfasermaterialien.

Gegenstand der Erfindung sind Stabilisatorgemische enthaltend 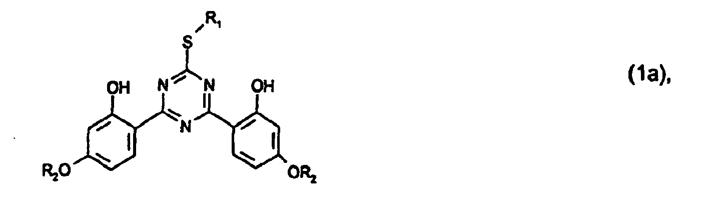 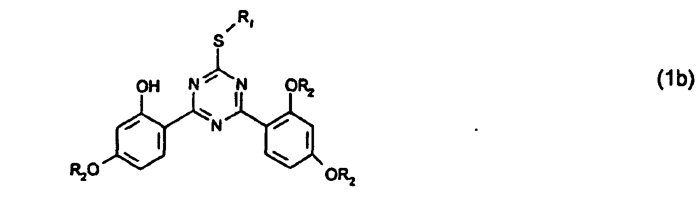 und 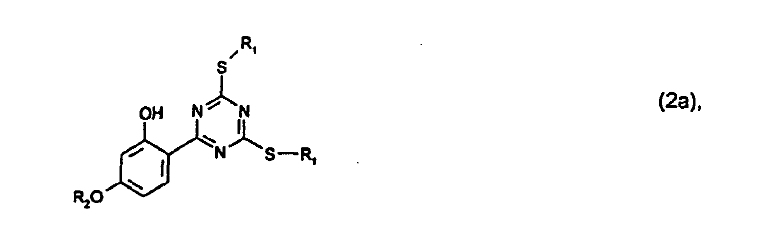 worin R₁ lineares oder verzweigtes C₁-C₁₈-Alkyl, C₃-C₈-Cycloalkyl, C₃-C₆-Alkenyl, gegebenenfalls substituiertes Aryl oder C₇-C₁₂-Aralkyl ist,
R₂ gegebenenfalls substituiertes lineares oder verzweigtes C₁-C₁₂-Alkyl, durch ein oder mehrere N-, S- oder O-Atome unterbrochenes lineares oder verzweigtes C₄-C₂₈-Alkyl, welches gegebenenfalls weitersubstituiert ist, oder einen Rest -CO-R₆ oder -SO₂-R₆ bedeutet, und R₆ C₁-C₁₂-Alkyl, gegebenenfalls durch C₁-C₄-Alkyl substituiertes Phenyl oder C₇-C₁₂-Aralkyl ist.

Bei R₁ als C₁-C₁₈-Alkyl handelt es sich um geradkettige oder verzweigte Alkylreste wie z.B. Methyl, Ethyl, n- oder iso-Propyl, n-, iso-, sec.- oder tert.-Butyl, 2-Ethylbutyl, n-oder iso-Pentyl, 1-Methylpentyl, 1,3-Dimethylbutyl, n-Hexyl, 1-Methylhexyl, n- oder iso-Heptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, Dodecyl, 1,1,3,3,5,5-Hexamethylhexyl. Tridecyl, Tetradecyl, Pentadecyl, Hexadecyl, Heptadecyl oder Octadecyl. R₁ steht als Alkylrest bevorzugt für C₁-C₆-Alkyl, besonders bevorzugt für C₁-C₄-Alkyl und insbesondere bevorzugt je für Methyl.

Bei R₁ als Cycloalkylrest handelt es sich z.B. um einen gesättigten 3- bis 8-gliedrigen carbocyclischen Ring, der gegebenenfalls durch eine oder mehrere C₁-C₄-Alkylgruppen, vorzugsweise Methylgruppen, substituiert ist. Beispiele sind Cyclopentyl, Cyclohexyl, Methylcyclohexyl oder Cycloheptyl. Vorzugsweise steht R₁ als Cycloalkylrest für unsubstituiertes oder durch 1-3 Methylgruppen substituiertes Cyclohexyl.

Beispiele für geeignete Alkenylreste R₁ sind Allyl, Isopropenyl, 2-Butenyl, 3-Butenyl, Isobutenyl, n-Penta-2,4-dienyl oder 3-Methyl-but-2-enyl. R₁ bedeutet als Alkenylrest bevorzugt Allyl oder Isopropenyl und besonders bevorzugt je Allyl.

R₁ steht als Arylrest z.B. für einen Biphenyl-, Naphthyl- oder insbesondere Phenylrest, wobei dieser jeweils z.B. durch C₁-C₄-Alkyl, C₁-C₄-Alkoxy oder Halogen weitersubstituiert sein kann. Bevorzugte Bedeutungen von R₁ als Arylrest ist unsubstituiertes oder durch C₁-C₄-Alkyl substituiertes Phenyl und insbesondere bevorzugt Phenyl.

Beispiele für geeignete Aralkylreste R₁ sind Benzyl, α-Methylbenzyl, Phenylethyl, Phenylpropyl, Phenylbutyl, Phenylpentyl oder Phenylhexyl. R₁ bedeutet als Aralkylrest bevorzugt Benzyl oder α-Methylbenzyl und besonders bevorzugt Benzyl.

Eine bevorzugte Ausführungsform der vorliegenden Erfindung betrifft Stabilisatorgemische enthaltend die Verbindungen der zuvor angegebenen Formeln, worin R₁ C₁-C₆-Alkyl, unsubstituiertes oder durch 1-3 Methylgruppen substituiertes Cyclohexyl, Allyl, Isopropenyl, unsubstituiertes oder durch C₁-C₄-Alkyl, C₁-C₄-Alkoxy oder Halogen substituiertes Phenyl, Benzyl oder α-Methylbenzyl bedeutet.

Eine besonders bevorzugte Ausführungsform der vorliegenden Erfindung betrifft Stabilisatorgemische enthaltend mindestens je eine Verbindung der zuvor angegebenen Formeln worin R₁ C₁-C₄-Alkyl, Cyclohexyl, Allyl, Phenyl oder Benzyl bedeutet.

Eine insbesonders bevorzugte Ausführungsform der vorliegenden Erfindung betrifft Stabilisatorgemische von Verbindungen der zuvor angegebenen Formeln, worin R₁ C₁-C₄-Alkyl, besonders Methyl, bedeutet.

Steht R₂ für einen C₁-C₁₂-Alkylrest, so kann dies z.B. einer der zuvor für R₁ genannten C₁-C₁₂-Alkylreste oder ein entsprechender C₁-C₁₂-Alkylrest, der z.B. durch Halogen, Cyano, Hydroxy, Amino, C₁-C₄-Alkoxycarbonyl, Carbamoyl, N-Mono- oder N,N-Di-C₁-C₄-Alkylcarbamoyl, Glycidyl oder Phenyl substituiert ist, sein. Geeignete C₁-C₄-Alkoxycarbonyl-Substituenten sind z.B. Methoxycarbonyl oder Ethoxycarbonyl. Unter Glycidyl ist der 2,3-Epoxypropylrest zu verstehen.

R₂ steht als Alkylrest bevorzugt für C₁-C₆-Alkyl, besonders bevorzugt für C₁-C₄-Alkyl und insbesondere bevorzugt für Methyl, Ethyl oder n- oder iso-Propyl.

Bedeutet R₂ einen durch Heteroatome unterbrochenen Alkylrest, so handelt es sich z.B. um geradkettiges oder verzweigtes C₄-C₂₈-Alkyl, welches durch eine oder mehrere Gruppen -O-, -NH- oder -S- unterbrochen und gegebenenfalls durch Hydroxy oder einen Rest -OR₆, worin R₆ die zuvor angegebene Bedeutung hat, weitersubstituiert ist; anwesende Heteroatomgruppen -O-, -NH- oder -S- treten dabei nicht benachbart auf. Vorzugsweise bedeutet R₂ als durch Heteroatome unterbrochener Alkylrest einen geradkettigen oder verzweigten C₄-C₂₈-Alkylrest, welcher durch eine oder mehrere Gruppen -O- unterbrochen und durch Hydroxy oder einen Rest -OR₆ substituiert ist. R₂ entspricht als Heteroalkylrest bevorzugt der Formel -(CH₂CHR₇-O)ₙ-H, worin R₇ Methyl oder insbesondere Wasserstoff und n eine ganze Zahl von 1 bis 9 bedeuten.

Steht R₆ für einen C₁-C₁₂-Alkylrest, so kann dies z.B. einer der zuvor für R₁ genannten C₁-C₁₂-Alkylreste sein. R₆ bedeutet als Alkylrest bevorzugt C₁-C₄-Alkyl, besonders bevorzugt Methyl oder Ethyl und insbesondere bevorzugt Methyl. Bedeutet R₆ einen Phenylrest, handelt es sich vorzugsweise um Phenyl, o-, m- oder p-Tolyl und besonders bevorzugt um Phenyl. Bedeutet R₆ einen Aralkylrest, handelt es sich bevorzugt um Benzyl.

R₂ steht bevorzugt für C₁-C₆-Alkyl oder für geradkettiges oder verzweigtes C₄-C₂₈-Alkyl, welches durch ein oder mehrere Gruppen -O- unterbrochen und durch Hydroxy oder einen Rest -OR₆, worin R₆ C₁-C₄-Alkyl, Phenyl, o-, m- oder p-Tolyl oder Benzyl ist, substituiert ist, oder für unsubstituiertes oder durch C₁-C₄-Alkyl substituiertes Benzoyl oder Phenylsulfonyl. Besonders bevorzugte Bedeutungen von R₂ sind C₁-C₄-Alkyl, ein Rest der Formel -(CH₂CHR₇-O)ₙ-H, worin R₇ Methyl oder insbesondere Wasserstoff und n eine ganze Zahl von 1 bis 9 bedeuten, und Benzoyl. R₂ steht insbesondere bevorzugt für Methyl, Ethyl oder n- oder iso-Propyl.

Die Verbindungen der Formel (1) sind z.B. aus der EP-A-0584044 bekannt oder können nach den dort beschriebenen Verfahren, z.B. durch Kondensation von 1 Moläquivalent Cyanurchlorid mit ca. 1 Moläquivalent einer Verbindung R₁-SH, worin R₁ die zuvor angegebene Bedeutung hat, und Weiterreaktion des Kondensationsprodukts mit zwei Moläquivalenten der entsprechenden Benzolverbindung in Gegenwart einer Lewis-Säure, hergestellt werden.

Die Verbindungen der Formel (2a) sind zum Teil neu. Ein weiterer Gegenstand der Erfindung betrifft daher Verbindungen der zuvor angegebenen Formel (2), worin R₁ unabhängig voneinander je lineares oder verzweigtes C₁-C₁₈-Alkyl, C₃-C₈-Cycloalkyl, C₃-C₆-Alkenyl oder C₇-C₁₂-Aralkyl ist, und R₂

gegebenenfalls substituiertes lineares oder verzweigtes C₁-C₁₂-Alkyl, durch ein oder mehrere N-, S- oder O-Atome unterbrochenes lineares oder verzweigtes C₄-C₂₈-Alkyl, welches gegebenenfalls weitersubstituiert ist, oder einen Rest -CO-R₆ oder -SO₂-R₆ bedeuten, und R₆ C₁-C₁₂-Alkyl, gegebenenfalls durch C₁-C₄-Alkyl substituiertes Phenyl oder C₇-C₁₂-Aralkyl ist.

Die Verbindungen der Formel (2a) können in Analogie zu den bekannten Verbindungen der Formel (1) hergestellt werden, z.B. indem man 1 Moläquivalent Cyanurchlorid mit ca 1 Moläquivalent einer Verbindung R₁-SH und ca. 1 Moläquivalent einer Verbindung R₁'-SH umsetzt und in einem Folgeschritt mit ca. 1 Moläquivalent der entsprechenden Benzolverbindung in Gegenwart einer Lewis-Säure, vorzugsweise Aluminiumchlorid, umsetzt, wobei R₁ und R₁' jeweils die zuvor angegebene Bedeutung haben und verschieden oder vorzugsweise gleich sind.

Die erfindungsgemässen Stabilisatorgemische können nach an sich bekannten Methoden aus den Einzelverbindungen erhalten werden, z.B. durch Vermischen, gemeinsames Vermahlen oder gemeinsames Kristallisieren. Auch ein Vermischen durch gleichzeitiges oder nacheinander erfolgendes Einarbeiten der Verbindungen der Formeln (1) und (2) in das zu stabilisierende Textilfasersubstrat ist möglich.

Ein weiterer Gegenstand der Erfindung betrifft die Herstellung der erfindungsgemässen Stabilisatorgemische durch gemeinsame Synthese, was insbesondere für solche mindestens je eine Verbindung der zuvor angegebenen Formeln (1a), (1b) und (2a) enthaltenden Gemische bevorzugt ist, worin die Reste R₁ identisch sind. Danach werden die erfindungsgemässen Stabilisatorgemische z.B. hergestellt, indem man
(i) ein Cyanurhalogenid, z.B. Cyanurfluorid oder vorzugsweise Cyanurchlorid, in wässriger oder wässrig-organischer Lösung mit einem Ueberschuss einer Verbindung der Formel

   R₁ -S-X (3)

   worin X Wasserstoff oder ein Kation ist und R₁ die zuvor angegebene Bedeutung hat, gegebenenfalls in Gegenwart eines Halogenwasserstoffakzeptors, welcher in Fall von X = Wasserstoff anwesend sein muss, kondensiert,
(ii) das gemäss (i) erhältliche die Verbindungen der Formeln 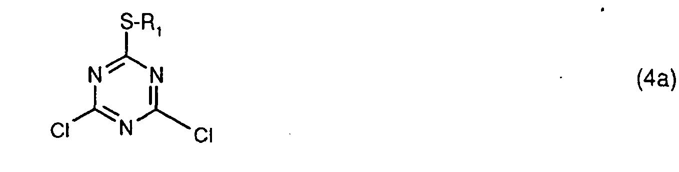 und 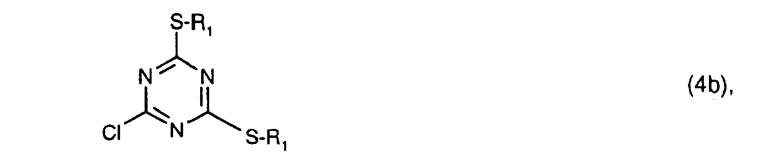 enthaltende Gemisch in Gegenwart eine Lewis-Säure mit Resorcin umsetzt, und
(iii) das gemäss (ii) erhältliche die Verbindungen der Formeln 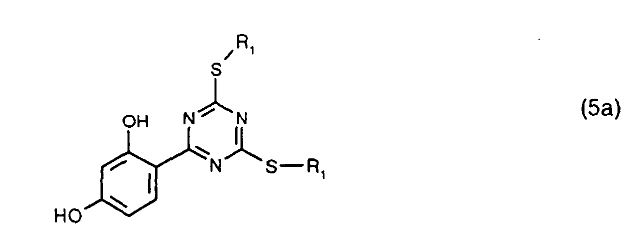 und 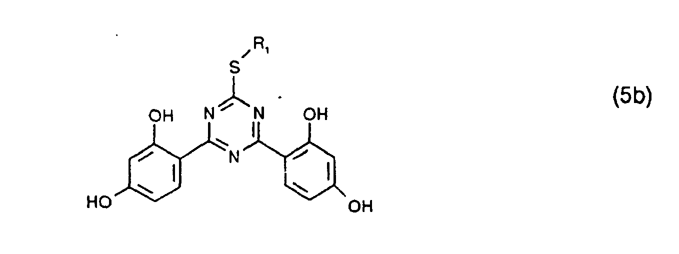
enthaltende Gemisch gegebenenfalls mit einem oder mehreren die Reste R₂ enthaltenden Alkylierungs- oder Acylierungsmitteln in ein erfindungsgemässes Stabilisatorgemisch überführt.

Die Reaktion der Verbindung der Formel (3) mit dem Cyanurhalogenid im Schritt (i) ist z.B. aus der GB-A-1,176,770 bekannt oder kann in Analogie dazu ausgeführt werden. Man führt die Umsetzung z.B. bei einer Temperatur von -5 bis 100°C und vorzugsweise 0 bis 50°C in einem Wasser und gegebenenfalls ein organisches Lösungsmittel, z.B. ein Alkylbenzol wie z.B. Toluol, Xylol, ein substituiertes Benzol wie z.B. Chlorbenzol, Nitrobenzol oder Anisol oder einen gegebenenfalls substituierten aliphatischen oder cycloaliphatischen Kohlenwasserstoff wie z.B. Pentan, Hexan, Cyclohexan, Dibutylether, Methylenchlorid, Chloroform, enthaltenden Medium durch. Bevorzugt als organisches Lösungsmittel sind Alkylbenzole, insbesondere Toluol, o-, m- oder p-Xylol oder ein Gemisch verschiedener Xylole. Geeignete Halogenwasserstoffakzeptoren im Schritt (i) sind z.B. Alkali- oder Erdalkalimetallcarbonate, -bicarbonate oder -hydroxide und vorzugsweise Alkalimetallhydroxide wie Natrium- oder Kaliumhydroxid. Die Zugabe eines Halogenwasserstoffakzeptors kann entfallen, wenn eine Verbindung der Formel (3a) eingesetzt wird, worin X ein Kation, z.B. ein Alkalimetall-, Erdalkalimetall- oder Ammoniumkation und vorzugsweise ein Alkalimetall-Kation wie das Kalium- oder insbesondere das Natrium-Kation, ist. Das Mengenverhältnis, in dem die Verbindungen der Formeln (4a) und (4b) im Schritt (i) anfallen, hängt z.B. vom stöchiometrischen Verhältnis der eingesetzten Reaktanden, dem Cyanurhalogenid und der Verbindung der Formel (3), ab, und kann z.B. zwischen 95 und 5 Gew.-% Verbindung der Formel (4a) und 5 und 95 Gew.-% Verbindung der Formel (4b) variieren.

Die Reaktion des gemäss (i) erhältlichen Produkts mit Resorcin findet im allgemeinen unter den für Friedel-Crafts-Reaktionen üblichen Bedingungen, z.B. bei einer Temperatur von 10 bis 50°C und vorzugsweise 25 bis 40°C in Gegenwart einer Lewis-Säure, vorteilhaft Aluminiumchlorid, statt. Das Resorcin und die Lewissäure sind dabei jeweils vorteilhaft in mindestens äquimolaren Mengen oder vorzugsweise in einem gewissen molaren Ueberschuss, bezogen auf die molare Menge des am Cyanurhalogenid zu ersetzenden Halogen, anwesend. Beispielsweise hat sich sowohl für das molare Verhältnis von Resorcin/zu ersetzendem Halogen wie auch für das molare Verhältnis von Lewissäure/zu ersetzendem Halogen jeweils ein Wert von 1:1 bis 2:1 und vorzugsweise 1,1:1 bis 1,5:1 als günstig erwiesen.

Die Schritte (i) und (ii) des erfindungsgemässen Verfahrens können getrennt oder auch als Eintopf-Reaktion ausgeführt werden. Bei der bevorzugten Ausführung mittels Eintopf-Verfahren wird vorteilhaft die im Schritt (i) anfallende organische Phase z.B. mittels Azeotropdestillation getrocknet und ohne weitere Reinigung gemäss Schritt (ii) weiter umgesetzt.

Geeignete Alkylierungsmittel im fakultativen Schritt (iii) sind z.B. Alkylhalogenide R-Hal, worin R ein beliebiger Alkylrest und Hal Halogen, z.B. Chlor, Brom oder Jod, bedeutet, Dialkylcarbonate wie z.B. Dimethylcarbonat, Mono- oder Dialkylsulfate der allgemeinen Formel R-O-SO₂-OH, R-O-SO₂-O-R oder R-O-SO₂-C₆H₅-CH₃, Alkylphosphonate der Formel R-O-P(O)(Z)-O-R, worin R jeweils einen beliebigen Alkylrest darstellt und Z z.B. für C₁-C₄-Alkyl oder Hydroxy steht oder Amidacetale wie z.B. Dimethylformamid-dimethylacetal. Beispiele für Methylierungsmittel sind Dimethylsulfat, Methyltosylat oder Dimethylmethanphosphonat (DMMP). Die Einführung eines Ethylrestes R₂ erfolgt z.B. mit Diethylsulfat. Die Alkylierungsreaktion wird im allgemeinen bei Temperaturen von z.B. 10 bis 200°C und bevorzugt 80 bis 150°C, in Gegenwart einer Base, wie z.B. einem Alkalicarbonat oder Alkalihydroxid, z.B. Natriumcarbonat, Kaliumcarbonat oder Natriumhydroxid, mit einem Ueberschuss an Alkylierungsmittel durchgeführt, wobei z.B. die Stöchiometrie und die Reaktionszeit den Grad der Alkylierung beeinflussen. Die in den Verbindungen der Formeln (5a) und (5b) in p-Stellung zur Bindung an den Triazinylring befindlichen Hydroxygruppen sind im allgemeinen leichter zugänglich als diejenigen in o-Stellung und werden daher zuerst alkyliert. Geeignete Acylierungsmittel sind z.B. Verbindungen der Formel R₆-CO-Y oder R₆-SO₂-Y, worin R₆ die zuvor angegebene Bedeutung hat und Y Halogen, vorzugsweise Chlor, bedeutet.

Ein nach der erfindungsgemässen gemeinsamen Synthese erhaltenes Stabilisatorgemisch enthält im wesentlichen die Verbindungen der Formel 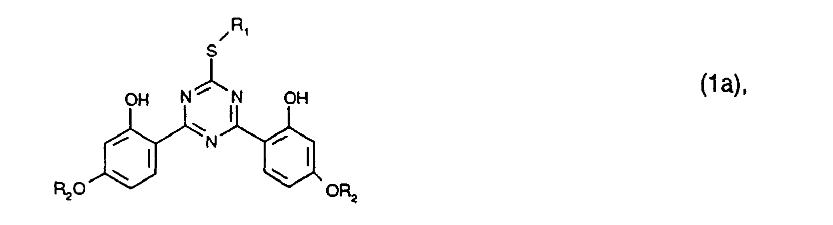 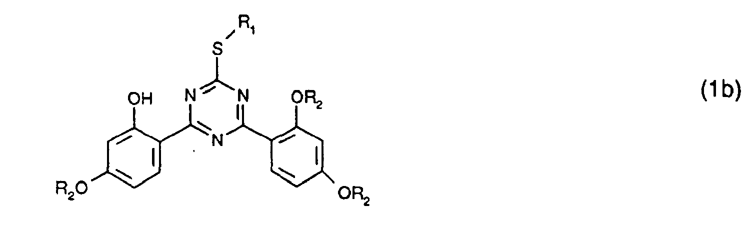 und 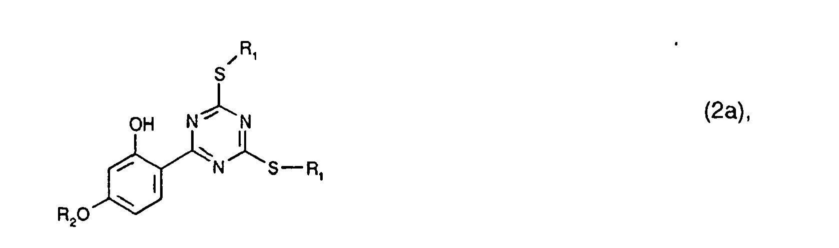 worin für R₁ und R₂ jeweils die zuvor angegebenen Bedeutungen und Bevorzugungen gelten. Besonders bevorzugt sind Stabilisatorgemische enthaltend 50 bis 95 Gew.-% Verbindungen der oben angegebenen Formeln (1a) und (1b) und 50 bis 5 Gew.-% der Verbindung der oben angegebenen Formel (2a).

Insbesondere bevorzugt sind Stabilisatorgemische enthaltend 50 bis 80 Gew.-% Verbindungen der oben angegebenen Formeln (1a) und (1b) und 50 bis 20 Gew.-% der Verbindung der oben angegebenen Formel (2a), worin R₁ C₁-C₄-Alkyl, Cyclohexyl, Allyl, Phenyl oder Benzyl und R₂ C₁-C₄-Alkyl, ein Rest der Formel -(CH₂CHR₄-O)ₙ-H, worin R₄ Methyl oder insbesondere Wasserstoff und n eine ganze Zahl von 1 bis 9 bedeuten, oder Benzoyl sind.

Die erfindungsgemässen Stabilisatorgemische sind verwendbar als Stabilisator für organische Materialien insbesondere gegen Schädigung durch Licht, Sauerstoff oder Hitze. Ganz besonders eignen sich die erfindungsgemässen Verbindungen und Stabilisatorgemische als Lichtstabilisatoren (UV-Absorber).

Besondere Vorteile der erfindungsgemässen Gemische sind unter anderem die hervorragende Beständigkeit des stabilisierten Materials gegen Einflüsse von Witterung und Licht, sowie die hervorragende Photostabilität der eingearbeiteten Stabilisatorgemische. Auch die ausgezeichnete Substratkompatibilität der erfindungsgemässen Gemische verdient Erwähnung. Insbesondere zeichnen sich die erfindungsgemässen Gemische durch einen hohen Aufziehgrad aus.

Die zu stabilisierenden Materialien können z.B. Oele, Fette, Wachse, Kosmetika oder Biocide sein. Von besonderem Interesse ist die Verwendung in polymeren Materialien, wie sie in Kunststoffen, Kautschuken, Anstrichstoffen, photographischen Materialien oder Klebstoffen vorliegen. Beispiele für Polymere und andere Substrate, die auf diese Weise stabilisiert werden können, sind die folgenden:
1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbomen; ferner Polyethylen (das gegebenenfalls vernetzt sein kann), z.B. Polyethylen hoher Dichte (HDPE), Polyethylen hoher Dichte und hoher Molmasse (HDPE-HMW), Polyethylen hoher Dichte und ultrahoher Molmasse (HDPE-UHMW), Polyethylen mittlerer Dichte (MDPE), Polyethylen niederer Dichte (LDPE), lineares Polyethylen niederer Dichte (LLDPE), verzweigtes Polyethylen niederer Dichte (VLDPE).
   Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:
   a) radikalisch (gewöhnlich bei hohem Druck und hoher Temperatur).
   b) mittels Katalysator, wobei der Katalysator gewöhnlich ein oder mehrere Metalle der Gruppe IVb, Vb, Vlb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder n- oder σ-koordiniert sein können. Diese Metallkomplexe können frei oder auf Träger fixiert sein, wie beispielsweise auf aktiviertem Magnesiumchlorid, Titan(lll)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen la, lla und/oder llla sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (lonomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.
4. Kohlenwasserstoffharze (z.B. C₅-C₉) inklusive hydrierte Modifikationen davon (z.B. Klebrigmacherharze) und Mischungen von Polyalkylenen und Stärke.
5. Polystyrol, Poly-(p-methylstyrol), Poly-(α-methylstyrol).
6. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.
7. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.
8. Halogenhaltige Polymere, wie z.B. Polychloropren, Chlorkautschuk, chloriertes und bromiertes Copolymer aus lsobutylen-lsopren (Halobutylkautschuk), chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
9. Polymere, die sich von α,β-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.
10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
12. Homo- und Copolymere von cyclischen Ethern, wie Polyalkylenglyköle, Polyethylenoxyd, Polypropylenoxyd oder deren Copolymere mit Bisglycidylethern.
13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
14. Polyphenylenoxide und -sulfide und deren Mischungen mit Styrolpolymeren oder Polyamiden.
15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-mphenylen-isophthalamid. Block-Copolymere der vorstehend genanntenPolyamide mit Polyolefinen, Olefin-Copolymeren, lonomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
17. Polyharnstoffe, Polyimide, Polyamid-imide, Polyetherimide, Polyesterimide, Polyhydantoine und Polybenzimidazole.
18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
   18a. Polyester, sauer modifiziert.
19. Polycarbonate und Polyestercarbonate.
20. Polysulfone, Polyethersulfone und Polyetherketone.
21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
22. Trocknende und nicht-trocknende Alkydharze.
23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
24. Vernetzbare Acrylharze, die sich von substituierten Acrylsäureestern ableiten, wie z.B. von Epoxyacrylaten, Urethan-acrylaten oder Polyester-acrylaten.
25. Alkydharze, Polyesterharze und Acrylatharze, die mit Melaminharzen, Harnstoffharzen, lsocyanaten, lsocyanuraten, Polyisocyanaten oder Epoxidharzen vernetzt sind.
26. Vernetzte Epoxidharze, die sich von aliphatischen, cycloaliphatischen, heterocyclischen oder aromatischen Glycidylverbindungen ableiten, z.B. Produkte von Bisphenol-A-diglycidylethem, Bisphenol-F-diglycidylethem, die mittels üblichen Härtern wie z.B. Anhydriden oder Aminen mit oder ohne Beschleunigern vernetzt werden.
27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO, PBT/PC/ABS oder PBT/PET/PC.

Die erfindungsgemäßen Zusammensetzungen können außer dem erfindungsgemäßen Verbindungsgemisch noch andere Stabilisatoren oder sonstige Zusätze enthalten, wie z.B. Antioxidantien, weitere Lichtschutzmittel, Metalldesaktivatoren, Phosphite oder Phosphonite. Beispiele hierfür sind die folgenden Stabilisatoren:

### 1. Antioxidantien

1.1. Alkylierte Monophenole, z.B. 2,6-Di-tert-butyl-4-methylphenol, 2-Butyl-4,6-dimethylphenol, 2,6-Di-tert-butyl-4-ethylphenol, 2,6-Di-tert-butyl-4-n-butylphenol, 2,6-Di-tert-butyl-4-isobutylphenol, 2,6-Di-cyclopentyl-4-methylphenol, 2-(α-Methylcyclohexyly-4,6-dimethylphenol, 2,6-Di-octadecyl-4-methylphenol, 2,4,6-Tri-cyclohexylphenol, 2,6-Di-tert-butyl-4-methoxymethylphenol, lineare oder in der Seitenkette verzweigte Nonylphenole wie z.B. 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.
1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonlphenol.
1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis(3,5-di-tert-butyl-4-hydroxyphenyl)adipat.
1.4. Tocopherole, z.B. α-Tocopherol, β-Tocopherol, γ-Tocopherol, δ-Tocopherol und Mischungen davon (Vitamin E).
1.5. Hydroxylierte Thiodiphenylether, z.B. 2,2'-Thio-bis(6-tert-butyl-4-methylphenol), 2,2'-Thio-bis(4-octylphenol), 4,4'-Thio-bis(6-tert-butyl-3-methylphenol), 4,4'-Thio-bis-(6-tert-butyl-2-methylphenol), 4,4'-Thio-bis(3,6-di-sec.-amylphenol), 4,4'-Bis(2,6-dimethyl-4-hydroxyphenyl)-disulfid.
1.6. Alkyliden-Bisphenole, z.B. 2,2'-Methylen-bis(6-tert-butyl-4-methylphenol), 2,2'-Methylen-bis(6-tert-butyl-4-ethylphenol), 2,2'-Methylen-bis[4-methyl-6-(α-methylcyclohexyl)-phenol], 2,2'-Methylen-bis(4-methyl-6-cyclohexylphenol), 2,2'-Methylen-bis(6-nonyl-4-methylphenol), 2,2'-Methylen-bis(4,6-di-tert-butylphenol), 2,2'-Ethyliden-bis(4,6-di-tert-butylphenol), 2,2'-Ethyliden-bis(6-tert-butyl-4-isobutylphenol), 2,2'-Methylen-bis[6-(α-methylbenzyl)-4-nonylphenol], 2,2'-Methylen-bis[6-(α,α-dimethylbenzyl)-4-nonylphenol], 4,4'-Methylen-bis(2,6-di-tert-butylphenol), 4,4'-Methylen-bis(6-tert-butyl-2-methylphenol), 1,1-Bis(5-tert-butyl-4-hydroxy-2-methylphenyl)-butan, 2,6-Bis(3-tert-butyl-5-methyl-2-hydroxybenzyl)-4-methylphenol, 1,1,3-Tris(5-tert-butyl-4-hydroxy-2-methylphenyl)-butan, 1,1-Bis(5-tert-butyl-4-hydroxy-2-methyl-phenyl)-3-n-dodecylmercaptobutan, Ethylenglycol-bis[3,3-bis(3'-tert-butyl-4'-hydroxyphenyl)-butyrat], Bis(3-tert-butyl-4-hydroxy-5-methyl-phenyl)-dicyclopentadien, Bis[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis(3,5-di-tert-butyl-4-hydroxyphenyl)-propan, 2,2-Bis(5-tert-butyl-4-hydroxy-2-methylphenyl)-4-n-dodecylmercapto-butan, 1,1,5,5-Tetra-(5-tert-butyl-4-hydroxy-2-methylphenyl)-pentan.
1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tridecyl-4-hydroxy-3,5-di-tert-butylbenzyl-mercaptoacetat, Tris(3,5-di-tert-butyl-4-hydroxybenzyl)-amin, Bis(4-tert-butyl-3-hydroxy-2,6-dimethylbenzyl)-dithioterephthalat, Bis(3,5-di-tert-butyl-4-hydroxybenzyl)-sulfid, Isooctyl-3,5-di-tert-butyl-4-hydroxybenzyl-mercaptoacetat.
1.8. Hydroxybenzylierte Malonate, z.B. Dioctadecyl-2,2-bis(3,5-di-tert-butyl-2-hydroxybenzyl)-malonat, Di-octadecyl-2-(3-tert-butyl-4-hydroxy-5-methylbenzyl)-malonat, Di-dodecyl-mercaptoethyl-2,2-bis(3,5-di-tert-butyl-4-hydroxybenzyl)-malonat, Di-[4-(1,1,3,3-tetramethylbutyl)-phenyl]-2,2-bis(3,5-di-tert-butyl-4-hydroxybenzyl)-malonat.
1.9. Hydroxybenzyl-Aromaten, z.B. 1,3,5-Tris(3,5-di-tert-butyl-4-hydroxybenzyl)-2,4,6-trimethylbenzol, 1,4-Bis(3,5-di-tert-butyl-4-hydroxybenzyl)-2,3,5,6-tetramethylbenzol, 2,4,6-Tris-(3,5-di-tert-butyl-4-hydroxybenzyl)-phenol.
1.10. Triazinverbindungen, z.B. 2,4-Bis-octylmercapto-6-(3,5-di-tert-butyl-4-hydroxyanilino)-1,3,5-triazin, 2-Octylmercapto-4,6-bis(3,5-di-tert-butyl-4-hydroxyanilino)-1,3,5-triazin, 2-Octylmercapto-4,6-bis(3,5-di-tert-butyl-4-hydroxyphenoxy)-1,3,5-triazin, 2,4,6-Tris(3,5-di-tert-butyl-4-hydroxyphenoxy)-1,2,3-triazin, 1,3,5-Tris(3,5-di-tert-butyl-4-hydroxybenzyl)-isocyanurat, 1,3,5-Tris(4-tert-butyl-3-hydroxy-2,6-dimethylbenzyl)-isocyanurat, 2,4,6-Tris(3,5-di-tert-butyl-4-hydroxyphenylethyl)-1,3,5-triazin, 1,3,5-Tris(3,5-di-tert-butyl-4-hydroxyphenyl-propionyl)-hexahydro-1,3,5-triazin, 1,3,5-Tris(3,5-dicyclohexyl-4-hydroxybenzyl)-isocyanurat.
1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.
1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.
1.13. Ester der β-(3,5-Di-tert-butyl-4-hydroxyphenyl)-propionsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
1.14. Ester der β-(5-tert-Butyl-4-hydroxy-3-methylphenyl)-propionsäure mit ein- oder mehrwertigen Alkoholen, wie z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
1.15. Ester der β-(3,5-Dicyclohexyl-4-hydroxyphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N,N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)-isocyanurat, N, N'-Bis(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.
1.17. Amide der β-(3,5-Di-tert-butyl-4-hydroxyphenyl)-propionsäure, wie z.B. N,N'-Bis(3,5-di-tert-butyl-4-hydroxyphenylpropionyl)-hexamethylendiamid, N,N'-Bis(3,5-di-tert-butyl-4-hydroxyphenylpropionyl)-trimethylendiamid, N,N'-Bis(3,5-di-tert-butyl-4-hydroxyphenylpropionyl)-hydrazid, N,N'-Bis[2-(3-[3,5-di-tert-butyl-4-hydroxyphenyl]-propionyloxy)ethyl]oxamid (Naugard^{®}XL-1 der Firma Uniroyal).
1.18. Ascorbinsäure (Vitamin C).
1.19. Aminische Antioxidantien, wie z.B. N,N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(2-naphthyl)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-lsopropoxy-diphenylamin, N-Phenyl-1-naphthylamin, N-(4-tert-Octylphenyl)-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylamino-phenol, 4-Dodecanoylamino-phenol, 4-Octadecanoylaminophenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Nonyldiphenylaminen, Gemisch aus mono- und dialkylierten Dodecyldiphenylaminen, Gemisch aus mono- und dialkylierten Isopropyl/Isohexyl-diphenylaminen, Gemische aus mono- und dialkylierten tert-Butyldiphenylaminen, 2,3-Dihydro-3,3-dimethyl-4H-1,4-benzothiazin, Phenothiazin, Gemisch aus mono- und dialkylierten tert-Butyl/tert-Octyl-phenothiazinen, Gemisch aus mono- und dialkylierten tert-Octyl-phenothiazinen, N-Allylphenothiazin, N,N,N',N'-Tetraphenyl-1,4-diaminobut-2-en, N,N-Bis-(2,2,6,6-tetramethyl-piperidin-4-yl-hexamethylendiamin, Bis-(2,2,6,6-tetramethylpiperidin-4-yl)-sebacat, 2,2,6,6-Tetramethypiperidin-4-on, 2,2,6,6-Tetramethylpiperidin-4-ol.

### 2. UV-Absorber und Lichtschutzmittel

2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis(α,α-dimethylbenzyl)-2'-hydroxyphenyl)-benzotriazol, 2-(3'-tert-Butyl-2'-hydroxy-5'-(2-octyloxycarbonylethyl)phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)-phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300; 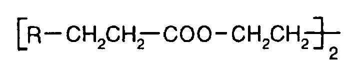 mit R = 3'-tert-Butyl-4'-hydroxy-5'-2H-benzotriazol-2-yl-phenyl; 2-[2'-Hydroxy-3'-(α,α-dimethylbenzyl)-5'-(1,1,3,3-tetramethylbutyl)-phenyl]-benzotriazol; 2-[2'-Hydroxy-3'-(1,1,3,3-tetramethylbutyl)-5'-(α,α-dimethylbenzyl)-phenyl]-benzotriazol.
2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.
2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.
2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.
2.5. Nickelverbindungen, wie z.B. Nickelkomplexe des 2,2'-Thio-bis[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
2.6. Sterisch gehinderte Amine, wie z.B. Bis(2,2,6,6-tetramethyl-piperidin-4-yl)-sebacat, Bis-(2,2,6,6-tetramethyl-piperidin-4-yl)-succinat, Bis(1,2,2,6,6-pentamethylpiperidin-4-yl)-sebacat, Bis(1-octyloxy-2,2,6,6-tetramethylpiperidin-4-yl)-sebacat, n-Butyl-3,5-di-tert-butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, lineare oder cyclische Kondensationsprodukte aus N,N'-Bis(2,2,6,6-Tetramethyl-4-piperidyl)-hexamethylendiamin und 4-tert-Octylamino-2,6-dichlor-1,3,5-s-triazin, Tris(2,2,6,6-tetramethyl-4-piperidyl)-nitrilotriacetat, Tetrakis(2,2,6,6-tetramethyl-4-piperidyl)-1,2,3,4-butantetraoat, 1,1'-(1,2-Ethandiyl)-bis(3,3,5,5-tetramethyl-piperazinon), 4-Benzoyl-2,2,6,6-tetramethylpiperidin, 4-Stearyloxy-2,2,6,6-tetramethylpiperidin, Bis(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]-decan-2,4-dion, Bis(1-octyloxy-2,2,6,6-tetramethylpiperidyl)-sebacat, Bis(1-octyloxy-2,2,6,6-tetramethylpiperidyl)-succinat, lineare oder cyclische Kondensationsprodukte aus N,N'-Bis (2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis(3-aminopropylamino)ethan, Kondensationsprodukt aus 2-Chlor-4,6-di-(4-n-butylamino-1,2,2,6,6-pentamethylpiperidyl)-1,3,5-triazin und 1,2-Bis(3-aminopropylamino)-ethan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion, Gemisch von 4-Hexadecyloxy- und 4-Stearyloxy-2,2,6,6-tetramethylpiperidin, Kondensationsprodukt aus N,N'-Bis(2,2,6,6-tetramethyl-4-piperidyl)-hexamethylendiamin und 4-Cyclohexylamino-2,6-dichlor-1,3,5-triazin, Kondensationsprodukt aus 1,2-Bis(3-aminopropylamino)-ethan und 2,4,6-trichlor-1,3,5-triazin sowie 4-Butylamino-2,2,6,6-tetramethyl-piperidin (CAS Reg. No. [136504-96-6]); N-(2,2,6,6-tetramethyl-4-piperidyl)-n-dodecylsuccinimid, N-(1,2,2,6,6-pentamethyl-4-piperidyl)-n-dodecylsuccinimid, 2-Undecyl-7,7,9,9-tetramethyl-1-oxa-3,8-diaza-4-oxo-spiro[4,5]decan, Umsetzungsprodukt von 7,7,9,9-Tetramethyl-2-cycloundecyl-1-oxa-3,8-diaza-4-oxospiro-[4,5]decan und Epichlorhydrin, 1,1-Bis(1,2,2,6,6-pentamethyl-4-piperidyloxycarbonyl)-2-(4-methoxyphenyl)-ethen, N,N'-Bis-formyl-N,N'-bis(2,2,6,6-tetramethyl-4-piperidyl)-hexamethylendiamin, Diester der 4-Methoxy-methylen-malonsäure mit 1,2,2,6,6-Pentamethyl-4-hydroxy-piperidin, Poly[methylpropyl-3-oxy-4-(2,2,6,6-tetramethyl-4-piperidyl)]-siloxan, Reaktionsprodukt aus Maleinsäureanhydrid-α-olefin-copolymer und 2,2,6,6-Tetramethyl-4-aminopiperidin oder 1,2,2,6,6-Pentamethyl-4-aminopiperidin.
2.7. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'-di-tert-butyl-oxanilid, 2-Ethoxy-2'-ethyl-oxanilid, N,N'-Bis(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.
2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-(2-Hydroxy-4-tridecyloxyphenyl)-4,6-bis(2,4-dimethylphenyl)-1,3,5-triazin, 2-[2-Hydroxy-4-(2-hydroxy-3-butyloxy-propyloxy)phenyl]-4,6-bis(2,4-dimethylphenyl)-1,3,5-triazin, 2-[2-Hydroxy-4-(2-hydroxy-3-octyloxy-propyloxy)phenyl]-4,6-bis(2,4-dimethylphenyl)-1,3,5-triazin, 2-[4-(dodecyloxy/tridecyloxy-2-hydroxypropoxy)-2-hydroxy-phenyl]-4,6-bis(2,4-dimethylphenyl)-1,3,5-triazin, 2-[2-Hydroxy-4-(2-hydroxy-3-dodecyloxy-propoxy)phenyl]-4,6-bis(2,4-dimethylphenyl)-1,3,5-triazin, 2-(2-Hydroxy-4-hexyloxy)phenyl-4,6-diphenyl-1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxy-propoxy)phenyl]-1,3,5-triazin, 2-(2-Hydroxyphenyl)-4-(4-methoxyphenyl)-6-phenyl-1 ,3, 5-triazin, 2-{2-Hydroxy-4-[3-(2-ethylhexyl-1-oxy)-2-hydroxypropyloxy]phenyl}-4,6-bis(2,4-dimethylphenyl)-1,3,5-triazin.
3. Metalldesaktivatoren, wie z.B. N,N'-Diphenyloxalsäurediamid, N-Salicylal-N'-salicyloyl-hydrazin, N,N'-Bis(salicyloyl)-hydrazin, N,N'-Bis(3,5-di-tert-butyl-4-hydroxyphenylpropionyl)-hydrazin, 3-Salicyloylamino-1,2,4-triazol, Bis(benzyliden)-oxalsäuredihydrazid, Oxanilid, lsophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.
4. Phosphite und Phosphonite, wie z.B. Triphenylphosphit, Diphenylalkylphosphite, Phenyldialkylphosphite, Tris(nonylphenyl)-phosphit, Trilaurylphosphit, Trioctadecylphosphit, Distearyl-pentaerythritdiphosphit, Tris(2,4-di-tert-butylphenyl)-phosphit, Diisodecylpentaerythrit-diphosphit, Bis(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis(2,4-di-tert-butyl-6-methylphenyl)-methylphosphit, Bis(2,4-di-tert-butyl-6-methylphenyl)-ethylphosphit, 2,2',2"-Nitrilo[triethyl-tris(3,3',5,5'-tetra-tert-butyl-1,1'-biphenyl-2,2'-diyl)-phosphit], 2-Ethylhexyl-(3,3',5,5'-tetra-tert-butyl-1,1'-biphenyl-2,2'-diyl)-phosphit.
   Besonders bevorzugt werden die folgenden Phosphite verwendet:
   Tris(2,4-di-tert-butylphenyl)-phosphit (Irgafos^{®}168, Ciba-Geigy), Tris(nonylphenyl)-phosphit, 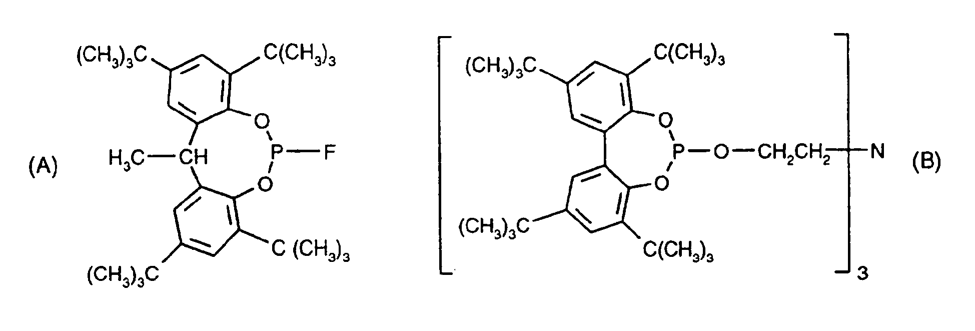 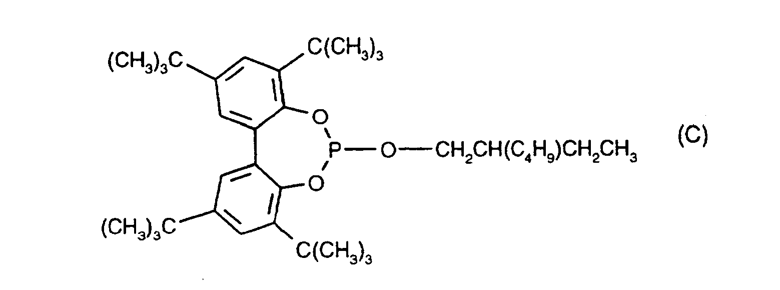 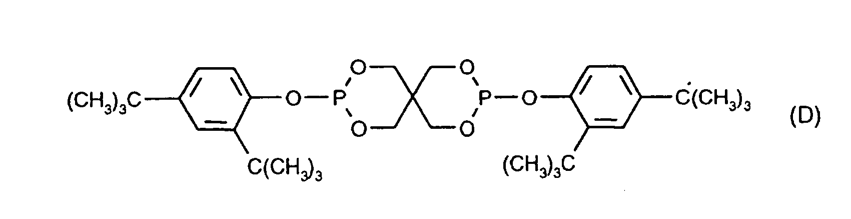 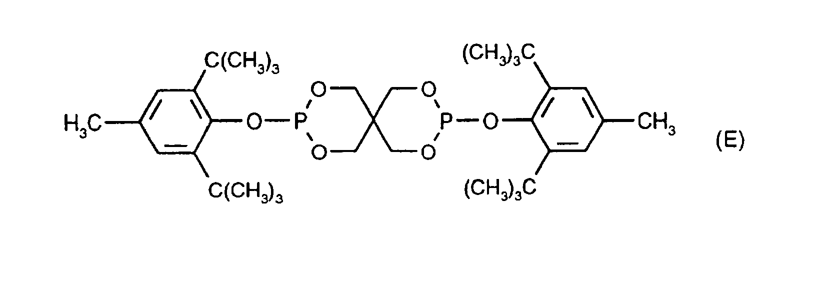 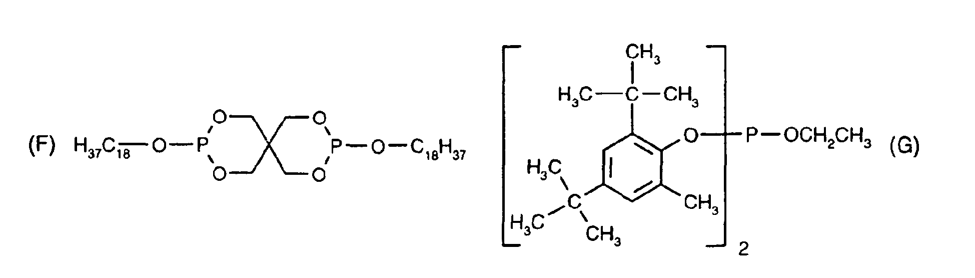
5. Hydroxylamine wie z.B. N,N-Dibenzylhydroxylamin, N,N-diethylhydroxylamin, N,N-Dioctylhydroxylamin, N,N-Dilaurylhydroxylamin, N,N-Ditetradecylhydroxylamin, N,N-Dihexadecylhydroxylamin, N,N-Dioctadecylhydroxylamin, N-Hexadecyl-N-octadecylhydroxylamin, N-Heptadecyl-N-octadecylhydroxylamin, N,N-Dialkylhydroxylamin aus hydrierten Talgfettaminen.
6. Nitrone wie z.B. N-Benzyl-alpha-phenyl-nitron, N-Ethyl-alpha-methyl-nitron, N-Octyl-alpha-heptyl-nitron, N-Lauryl-alpha-undecyl-nitron, N-Tetradecyl-alpha-tridecyl-nitron, N-Hexadecyl-alpha-pentadecyl-nitron, N-Octadecyl-alpha-heptadecyl-nitron, N-Hexadecyl-alpha-heptadecyl-nitron, N-Octadecyl-alpha-pentadecyl-nitron, N-Heptadecyl-alpha-heptadecyl-nitron, N-Octadecyl-alpha-hexadecyl-nitron, Nitrone abgeleitet von N,N-Dialkylhydroxylaminen hergestellt aus hydrierten Talgfettaminen.
7. Thiosynergisten wie z.B. Thiodipropionsäure-di-laurylester oder Thiodipropionsäure-distearylester.
8. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis(β-dodecylmercapto)-propionat.
9. Polyamidstabilisatoren, wie z.B. Kupfersalze in Kombination mit Jodiden und/oder Phosphorverbindungen und Salze des zweiwertigen Mangans.
10. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinkbrenzcatechinat.
11. Nukleierungsmittel, wie z.B. anorganische Stoffe wie z.B. Talk, Metalloxide wie Titandioxid oder Magnesiumoxid, Phosphate, Carbonate oder Sulfate von vorzugsweise Erdalkalimetallen; organische Verbindungen wie Mono- oder Polycarbonsäuren sowie ihre Salze wie z.B. 4-tert-Butylbenzoesäure, Adipinsäure, Diphenylessigsäure, Natriumsuccinat oder Natriumbenzoat; polymere Verbindungen wie z.B. ionische Copolymerisate ("lonomere").
12. Füllstoffe und Verstärkungsmittel, wie z.B. Calciumcarbonat, Silikate, Glasfasern, Glaskugeln, Talk, Kaolin, Glimmer, Bariumsulfat, Metalloxide und -hydroxide, Ruß, Graphit, Holzmehl und Mehle oder Fasern anderer Naturprodukte, synthetische Fasern.
13. Sonstige Zusätze, wie z.B. Weichmacher, Gleitmittel, Emulgatoren, Pigmente, Rheologieadditive, Katalysatoren, Verlaufshilfsmittel, Optische Aufheller, Flammschutzmittel, Antistatika, Treibmittel.
14. Benzofuranone bzw. lndolinone, wie z.B. in U.S. 4,325,863; U.S. 4,338,244; U.S. 5,175,312, U.S. 5,216,052; U.S. 5,252,643; DE-A-4316611; DE-A-4316622; DE-A-4316876; EP-A-0589839 oder EP-A-0591102 beschrieben, oder 3-[4-(2-Acetoxy-ethoxy)phenyl]-5,7-di-tert-butyl-benzofuran-2-on, 5,7-Di-tert-butyl-3-[4-(2-stearoyloxyethoxy)phenyl]-benzofuran-2-on, 3,3'-Bis[5,7-di-tert-butyl-3-(4-[2-hydroxyethoxy]phenyl) benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxy-phenyl)-5,7-di-tert-butyl-benzofuran-2-on, 3-(3,4-Dimethylphenyl)-5,7-di-tert-butyl-benzofuran-2-on, 3-(2,3-Dimethylphenyl)-5,7-di-tert-butyl-benzofuran-2-on.

Die erfindungsgemässen Stabilisatorgemische sind ganz besonders zur photochemischen und thermischen Stabilisierung von ungefärbten, gefärbten oder bedruckten Textilfasermaterialien geeignet, welches einen weiteren Gegenstand der vorliegenden Erfindung darstellt. Ein entsprechendes Verfahren ist dadurch gekennzeichnet, dass man das Textilfasermaterial mit einem Stabilisatorgemisch enthaltend mindestens eine Verbindung der zuvor angegebenen Formel (1) und mindestens eine Verbindung der zuvor angegebenen Formel (2) behandelt.

Als zu behandelnde Textilfasermaterialien kommen in erster Linie polyester- oder celluloseacetat-haltige Fasermaterialien in Betracht. Unter Polyesterfasern sind z.B. Celluloseesterfasern, wie z.B. Cellulose-21/2-acetatfasern und -triacetatfasern, und insbesondere lineare Polyesterfasern, die gegebenenfalls sauer modifiziert sind, zu verstehen, die z.B. durch Kondensation von Terephthalsäure mit Ethylenglykol oder von lsophthalsäure oder Terephthalsäure mit 1,4-Bis(hydroxymethyl)-cyclohexan erhalten werden, sowie Fasern aus Mischpolymeren von Terephthal- und lsophthalsäure und Ethylenglykol. Uebliche Polyesterfasern in der Textilfaserindustrie bestehen insbesondere aus Terephthalsäure und Ethylenglykol.

Bei dem zu behandelnden Textilfasermaterial kann es sich auch um ein Mischgewebe aus Polyesterfasern und anderen Fasern, z.B. Polyacrylnitril/Polyester-, Polyamid/Polyester-, Polyester/Baumwolle-, PolyesterNiskose- oder Polyester/Wolle-Mischfasem handeln, die in üblicher Weise diskontinuierlich oder kontinuierlich gefärbt oder bedruckt werden.

Das Textilfasermaterial kann in verschiedenen Aufmachungsformen vorliegen, z.B. als Stückware wie Gewirke oder Gewebe, oder als Garn z.B. auf Kreuzspulen, Kettbäumen usw..

Gut geeignet für das erfindungsgemässe Verfahren sind ferner Textilgewebe im Oberbekleidungssektor, die zumindest teilweise lichtdurchlässig sind. Werden solche Textilien nach dem erfindungsgmässen Verfahren behandelt, kann dadurch das unter dem Oberbekleidungsstoff befindliche Hautgewebe vor dem schädigenden Einfluss der UV-Strahlung geschützt werden. Dies drückt sich dadurch aus, dass mit einem erfindungsgemässen Stabilisator oder Stabilisatorgemisch behandelte Textilfasermaterialien einen gegenüber dem unbehandelten Gewebe markant erhöhten Sonnenschutzfaktor (UPF = Ultraviolet Protection Factor) aufweisen können.

Der UPF wird definiert als Quotient aus schädlicher UV-Strahlendosis ohne Sonnenschutz und schädlicher UV-Strahlendosis mit Sonnenschutz. Dementsprechend stellt ein UPF auch einen Massstab für die Durchlässigkeit der unbehandelten und der mit einer erfindungsgemässen Verbindung bzw. einem erfindungsgemässen Verbindungsgemisch behandelten Fasermaterialien für UV-Strahlung dar. Die Bestimmung des UPF von textilen Fasermaterialien wird z.B. in der WO 94/04515 oder in J. Soc. Cosmet. Chem. 40, Seiten 127-133 (1989) erläutert und kann analog dazu erfolgen.

Die erfindungsgemässen Stabilisatorgemische werden in einer Menge von z.B. 0,01 bis 10 Gew.-%, vorzugsweise 0,1 bis 5 Gew.-%, bezogen auf das Gewicht des Fasermaterials, eingesetzt.

Die erfindungsgemässen Stabilisatorgemische sind in Wasser schwerlöslich und werden daher in dispergierter Form appliziert. Dazu werden sie z.B. mit einem entsprechenden Dispergator mit Hilfe von z.B. Quarzkugeln und einem Schnellrührgerät auf eine Feinheit, die den applikatorischen Bedingungen gerecht wird, gemahlen.

Als Dispergatoren für die Stabilisatorgemische kommen z.B. in Betracht:
- saure Ester oder deren Salze von Alkylenoxidaddukten, wie z.B. saure Ester oder deren Salze eines Polyadduktes von 4 bis 40 Mol Ethylenoxid an 1 Mol eines Phenols, oder Phosphorsäureester der Addukte von 6 bis 30 Mol Ethylenoxid an 1 Mol 4-Nonylphenol, 1 Mol Dinonylphenol oder besonders an 1 Mol von Verbindungen, die durch Anlagerung von 1 bis 3 Mol von gegebenenfalls substituierten Styrolen an 1 Mol Phenol hergestellt werden,
- Polystyrolsulfonate,
- Fettsäuretauride,
- alkylierte Diphenyloxid-mono- oder -di-sulfonate,
- Sulfonate von Polycarbonsäureestern,
- mit einer organischen Dicarbonsäure, oder einer anorganischen mehrbasischen Säure in einen sauren Ester übergeführte Anlagerungsprodukte von 1 bis 60, vorzugsweise 2 bis 30 Mol Ethylenoxid und/oder Propylenoxid an Fettamine, Fettamide, Fettsäuren oder Fettalkohole mit je 8 bis 22 Kohlenstoffatomen oder an drei- bis sechswertige Alkanole mit 3 bis 6 Kohlenstoffatomen,
- Ligninsulfonate, und ganz besonders
- Formaldehyd-Kondensationsprodukte wie z.B. Kondensationsprodukte von ligninsulfonaten und/oder Phenol und Formaldehyd, Kondensationsprodukte von Formaldehyd mit aromatischen Sulfonsäuren, wie Kondensationsprodukte von Ditolylethersulfonaten und Formaldehyd, Kondensationsprodukte von Naphthalinsulfonsäure und/oder Naphthol- oder Naphthylaminsulfonsäuren mit Formaldehyd, Kondensationsprodukte von Phenolsulfonsäuren und/oder sulfoniertem Dihydroxydiphenylsulfon und Phenolen bzw. Kresolen mit Formaldehyd und/oder Harnstoff sowie Kondensationsprodukte von Diphenyloxid-disulfonsäure-Derivaten mit Formaldehyd.

Handelt es sich bei dem zu stabilisierenden Textilfasermaterial um gefärbtes Textilfasermaterial, was bevorzugt ist, so kommen als Farbstoffe insbesondere in Wasser nur gering lösliche Dispersionsfarbstoffe in Betracht. Sie liegen deshalb in der Färbeflotte zum grössten Teil in Form einer feinen Dispersion vor. Sie können verschiedenen Farbstoffklassen angehören, beispielsweise den Acridon-, Azo-, Anthrachinon-, Cumarin-, Methin-, Perinon-, Naphthochinonimin-, Chinophthalon-, Styryl-, oder Nitrofarbstoffen. Es können auch Mischungen von Dispersionsfarbstoffen erfindungsgemäss eingesetzt werden.

Die Färbungen erfolgen aus wässriger Flotte nach einem kontinuierlichen oder diskontinuierlichen Verfahren. Beim diskontinuierlichen Verfahren (Ausziehverfahren) kann das Flottenverhältnis in einem weiten Bereich gewählt werden, z.B. 1:1 bis 1:100, vorzugsweise 1:6 bis 1:50. Die Temperatur, bei der gefärbt wird, beträgt mindestens 50°C und in der Regel ist sie nicht höher als 140°C. Vorzugsweise liegt sie im Bereich von 80 bis 135°C.

Bei kontinuierlichen Färbeverfahren werden die Färbeflotten, die neben den Farbstoffen gegebenenfalls weitere Hilfsmittel enthalten können, auf das Stückmaterial durch beispielsweise Fouladieren, Sprühen oder Pflatschen aufgebracht und mittels Thermofixier- oder HT-Dämpfprozessen entwickelt.

Lineare Polyesterfasern und Cellulosefasern färbt man vorzugsweise nach dem sogenannten Hochtemperaturverfahren in geschlossenen und druckbeständigen Apparaten bei Temperaturen >100°C, bevorzugt zwischen 110° und 135°C und gegebenenfalls unter Druck. Als geschlossene Gefässe eignen sich beispielsweise Zirkulationsapparaturen, wie Kreuzspul- oder Baumfärbeapparate, Haspelkufen, Düsen- oder Trommelfärbemaschinen, MuffFärbeapparate, Paddeln oder Jigger.

Cellulose-21/2-acetatfasern färbt man vorzugsweise bei Temperaturen von 80-85°C.

Werden die erfindungsgemässen Stabilisatorgemische in der Färbeapplikation eingesetzt, so erfolgt die Anwendung z.B. so, dass man das Fasermaterial zunächst mit diesen Stabilisatorgemischen behandelt und anschliessend die Färbung durchführt, oder vorzugsweise gleichzeitig das Fasermaterial mit einem Stabilisatorgemisch und dem Farbstoff im Färbebad behandelt. Die Applikation der Stabilisatorgemische kann jedoch auch nachträglich auf die fertig hergestellte Färbung mittels Thermofixierung, z.B. bei 190 bis 230°C in einem Zeitraum von 30 Sekunden bis 5 Minuten erfolgen. Möglich ist auch eine Vorbehandlung mit einem erfindungsgemässen Stabilisatorgemisch, wobei das Textilmaterial gleichzeitig flächenfixiert wird.

Die Färbeflotten können auch weitere Zusätze, wie z.B.Färbereihilfsmittel, Dispergiermittel, Carrier, Wollschutz- und Netzmittel sowie auch Entschäumer enthalten.

Die Färbebäder können des weiteren Mineralsäuren, wie z.B. Schwefelsäure oder Phosphorsäure, oder zweckmässigerweise organische Säuren, zum Beispiel aliphatische Carbonsäuren wie Ameisensäure, Essigsäure, Oxalsäure oder Zitronensäure und/oder Salze wie Ammoniumacetat, Ammoniumsulfat oder Natriumacetat enthalten. Die Säuren dienen vor allem der Einstellung des pH-Wertes der erfindungsgemäss verwendeten Flotten, der zwischen vorzugsweise 4 und 5 liegt.

Vorzugsweise lässt man das Fasermaterial während 5 Minuten bei 40 bis 80°C im Bad, das den Farbstoff, ein Stabilisatorgemisch und gegebenenfalls weitere Zusätze enthält und auf einen pH-Wert von 4,5 bis 5,5 eingestellt ist, vorlaufen, erhöht die Temperatur innerhalb von 10 bis 20 Minuten auf 125 bis 130°C und behandelt für 15 bis 90 Minuten, vorzugsweise 30 Minuten, bei dieser Temperatur weiter.

Die Fertigstellung der Färbungen erfolgt durch Abkühlen der Färbeflotte auf 50 bis 80°C, Spülen der Färbungen mit Wasser und gegebenenfalls durch Reinigung auf übliche Weise im alkalischen Medium unter reduktiven Bedingungen. Die Färbungen werden dann wiederum gespült und getrocknet. Bei Verwendung von Küpenfarbstoffen für den Celluloseanteil wird die Ware auf übliche Weise zuerst mit Hydrosulfit bei einem pH-Wert von 6 bis 12,5 und dann mit einem Oxidationsmittel behandelt und schliesslich ausgewaschen.

Für die Herstellung von Drucken werden die erfindungsgemässen Stabilisatorgemische in Form ihrer wässrigen Dispersionen den Druckpasten beigemischt. Die Druckpaste enthält dabei das entsprechende Stabilisatorgemisch in Mengen von z.B. 0,1 bis 10%, vorzugsweise 0,1 bis 5%, bezogen auf das Gewicht der Druckpaste.

Die Menge der Farbstoffe, die den Druckpasten zugesetzt werden, richtet sich nach der gewünschten Farbnuance; im allgemeinen haben sich Mengen von 0,01 bis 15, vorzugsweise 0,02 bis 10 Gewichtsprozent, bezogen auf das eingesetzte Textilmaterial, bewährt.

Die Druckpasten enthalten neben den Farbstoffen und der wässrigen Stabilisatorgemisch-Dispersion zweckmässigerweise säurestabile Verdickungsmittel, vorzugsweise natürlicher Herkunft wie Kernmehlabkömmlinge, insbesondere Natriumalginat für sich allein oder im Gemisch mit modifizierter Cellulose, insbesondere mit vorzugsweise 20 bis 25 Gewichtsprozent Carboxymethylcellulose. Daneben können die Druckpasten noch Säurespender, wie Butyrolacton oder Natriumhydrogenphosphat, Konservierungsmittel, Sequestriermittel, Emulgatoren, wasserunlösliche Lösungsmittel, Oxidationsmittel oder Entlüftungsmittel enthalten.

In Betracht kommen als Konservierungsmittel vor allem formaldehydabgebende Mittel, wie z.B. Paraformaldehyd oder Trioxan, vor allem wässrige, etwa 30 bis 40-gewichtsprozentige Formaldehydlösungen; als Sequestriermittel z.B. nitrilotriessigsaures Natrium, ethylendiamintetraessigsaures Natrium, vor allem Natrium-Polymetaphosphat, insbesondere Natrium-Hexametaphosphat; als Emulgatoren vor allem Addukte aus einem Alkylenoxid und einem Fettalkohol, insbesondere einem Addukt aus Oleylalkohol und Ethylenoxid; als wasserunlösliche Lösungsmittel hochsiedende, gesättigte Kohlenwasserstoffe, vor allem Paraffine mit einem Siedebereich von etwa 160 bis 210°C (sogenannte Lackbenzine); als Oxidationsmittel z.B. eine aromatische Nitroverbindung, vor allem eine aromatische Mono- oder Dinitrocarbonsäure oder -sulfonsäure, die gegebenenfalls als Alkylenoxidaddukt vorliegt, insbesondere eine Nitrobenzolsulfonsäure; und als Entlüftungsmittel z.B. hochsiedende Lösungsmittel, vor allem Terpentinöle, höhere Alkohole, vorzugsweise C₈- bis C₁₀-Alkohole, Terpenalkohole oder Entlüftungsmittel auf Basis von Mineral- und/oder Silikonölen, insbesondere Handelsformulierungen aus etwa 15 bis 25 Gewichtsprozent eines Mineral- und Silikonölgemisches und etwa 75 bis 85 Gewichtsprozent eines C₈-Alkohols wie z.B. 2-Ethyl-n-hexanol.

Beim Bedrucken der Fasermaterialien wird die Druckpaste ganzflächig oder stellenweise direkt auf das Fasermaterial aufgebracht, wobei zweckmässigerweise Druckmaschinen üblicher Bauart, z.B. Ink-Jet-Druck-, Vigoureuxdruck-, Tiefdruck-, Rotationssiebdruck- oder Flachfilmdruckmaschinen, eingesetzt werden.

Das Fasermaterial wird nach dem Bedrucken bei Temperaturen bis 150°C, vorzugsweise bei 80° bis 120°C getrocknet.

Anschliessend erfolgt die Fixierung des Materials durch eine Wärmebehandlung bei Temperaturen von vorzugsweise 100° bis 220°C. Die Wärmebehandlung erfolgt im allgemeinen mit überhitztem Wasserdampf unter Druck.

Je nach Temperatur kann die Fixierung 20 Sekunden bis 10 Minuten, vorzugsweise 4 bis 8 Minuten erfolgen.

Die Fertigstellung der Drucke erfolgt ebenfalls auf übliche Weise durch Spülen mit Wasser und kann gegebenenfalls durch zusätzliche Reinigung im alkalischen Medium unter reduktiven Bedingungen, z.B. mittels Natriumdithionit vorgenommen werden. Im letzteren Fall werden die Druckfärbungen wiederum gespült, entwässert und getrocknet.

Die mit den erfindungsgemässen Stabilisatorgemischen behandelten Textilfasern weisen eine gute Beständigkeit gegen die schädigenden Einflüsse von Licht, Sauerstoff und Wärme auf. Insbesondere lassen sich mittels des erfindungsgemässen Verfahrens hochlichtechte und sublimationsbeständige Polyester-Färbungen und Drucke erzielen. Eine gezielte Vor- oder Nachbehandlung des Fasermaterials ist mit dem erfindungsgemässen Verfahren nicht erforderlich.

In den folgenden Beispielen beziehen sich die Prozentsätze auf das Gewicht. Die Mengen beziehen sich bei den Farbstoffen und bei den UV-Absorbem auf Reinsubstanz.
Beispiel 1: Zu einem Gemisch von 541,4 g Cyanurchlorid in 1327 g Xylol (Isomerengemisch) werden unter Rühren 984 g Eis eingetragen. Anschliessend lässt man innerhalb von 10 min 1130 g einer 21 prozentigen wässerigen Lösung von Natriummethylmercaptid unter gutem Rühren zulaufen, wobei sich die Innentemperatur auf ca. 30°C erhöht. Man rührt noch während einer Stunde. Nach der Phasentrennung trennt man die untere wässerige Phase ab. Die organische Phase wird einmal mit 885 g Wasser gewaschen. Man erhält 2044 g xylolische Lösung, welche 18 Gew.-% 1-Methylthio-3,5-dichlor-s-triazin und 7,1 Gew.-% 1,3-Bis-methylthio-5-chlor-s-triazin enthält.
Beispiel 2: Zu 200 g der gemäss Beispiel 1 erhaltenen xylolischen Lösung enthaltend 36 g 1-Methylthio-3,5-dichlor-s-triazin und 14,2 g 1,3-Bis-methylthio-5-chlor-s-triazin werden bei 25°C unter gutem Rühren 63,71 g wasserfreies Aluminiumchlorid eingetragen. Das Gemisch wird während 15 min gerührt. Anschliessend lässt man innerhalb von 1,5 h eine Lösung von 52,64 g Resorcin in 110 g Sulfolan zum Reaktionsgemisch zugetropfen. Die Reaktionstemperatur wird dabei von anfangs 45°C bis auf ca. 50°C erhöht. Zuletzt rührt man zur Beendigung der Kondensation noch während 1,5 h bei 70°C und einer Stunde bei 80°C nach. Das emulsionsartige Gemisch wird auf 50°C gekühlt und der Friedel-Crafts-Komplex durch vorsichtiges Zutropfen eines Gemisches aus 62 g 32%iger Salzsäure und 455 g Wasser hydrolysiert. Danach wird das Xylol azeotrop abdestilliert und die Produktsuspension abgenutscht. Das Nutschgut wird mit heissem Wasser ausgewaschen und im Vakuum bei 80°C getrocknet. Man erhält 78,1 g eines Pulvers, das die Verbindungen der Formeln 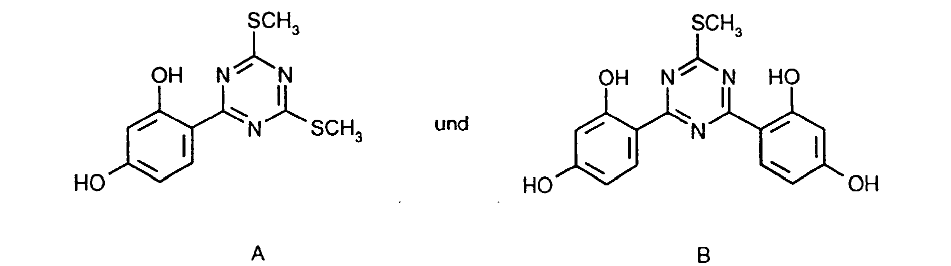 in einem Gewichtsverhältnis von A:B = 15:85 enthält.
Beispiel 3: Zu einer Anschlämmung von 20 g des gemäss Beispiel 2 erhältlichen Produktgemisches in 180 ml Methyläthylketon werden 60 ml 2N Natronlauge zugetropft. Anschliessend tropft man bei 33 bis 35°C innerhalb von einer Stunde 13,99 g Dimethylsulfat zum Gemisch. Man rührt während 16 h nach und neutralisiert das Produktgemisch mit 2N Salzsäure. Das organische Lösungsmittel wird durch azeotrope Destillation und die kristallinen Produkte schliesslich durch Filtration abgetrennt. Man wäscht den Filterrückstand mit Wasser nach und trocknet im Vakuum bei 70°C. Man erhält ein Pulver, welches die Verbindungen der Formeln C, D und E im Gewichtsverhältnis 71:13:16 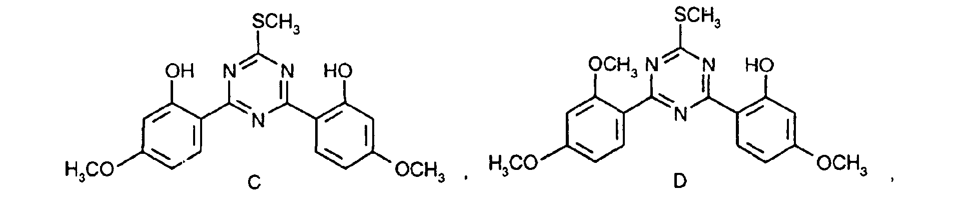 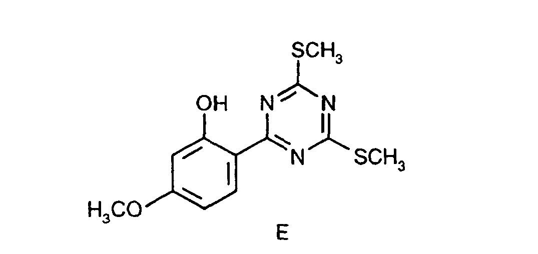 enthält.
Beispiel 4: Verfährt man wie in Beispiel 3 beschrieben, setzt jedoch anstelle von 13,99 g Dimethylsulfat 17,88 g Diethylsulfat ein und hält die Reaktionstemperatur bei 40°C, so erhält man ein Produktgemisch, das im wesentlichen die Verbindungen der Formeln 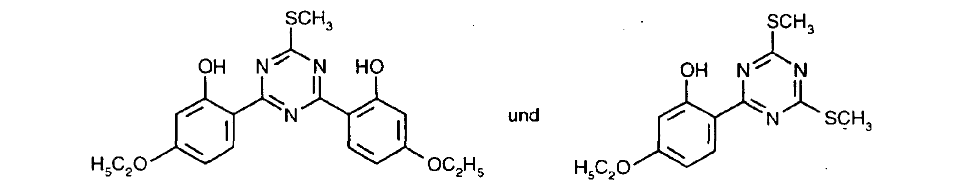 enthält.
Beispiel 5: Zu einer toluolischen Lösung von 28,7 g 1-Methylthio-3,5-dichlor-s-triazin (Herstellung gemäss GB-A-1,176,770, Beispiel 1) werden bei 80°C innerhalb einer Stunde 15,4 g Natriummethylmercaptid als 21proz. wässerige Lösung zugetropft. Anschliessend werden 50 ml Wasser zugegeben und während einer weiteren Stunde bei 90-95°C nachgerührt. Das Gemisch wird auf Raumtemperatur abgekühlt und mit Eis/Eiswasser und 100 ml 1N Natriumhydroxidlösung versetzt. Nach kurzem Verrühren trennt man die organische Phase ab, wäscht nochmals mit Wasser und trocknet über Calciumchlorid.
   Zu 121,5 g der erhaltenen toluolischen Lösung enthaltend 17,1 Gew.-% 1,3-Bis-methylthio-5-chlor-s-triazin werden 14,7 g wasserfreies Aluminium(lll)chlorid eingetragen. Dann tropft man bei einer Temperatur von 45 bis 50°C innerhalb von 25 min eine Lösung von 12,1 g Resorcin in 25 ml Sulfolan ein. Man rührt noch während 3 h bei 75°C nach. Die Emulsion wird auf ca. 60°C abgekühlt und der Friedel-Crafts-Komplex vorsichtig mit einem Gemisch aus 15 ml Konz. Salzsäure und 200 ml Wasser zersetzt. Toluol wird durch azeotrope Destillation abgetrennt und die Produktsuspension abgenutscht. Der Rückstand wird gut mit heissem Wasser ausgewaschen und abgepresst. Das Rohprodukt wird aus Wasser/Dioxan umkristallisiert. Man erhält die Verbindung der Formel 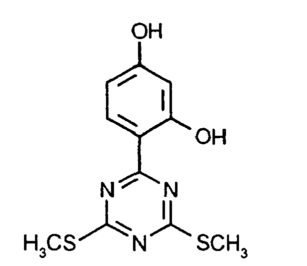 als Pulver.
Beispiel 6: Eine Anschlämmung von 8,43 g der Verbindung des Beispiels 5 in 27,9 g Dimethylmethylphosphonat wird mit 3,37 g Natriumcarbonat versetzt und während 2 3/4 h auf 120°C erhitzt. Das Reaktionsgemisch wird auf 75 bis 80°C abgekühlt und tropfenweise mit einer Mischung aus 70 ml Methanol und 20 ml Wasser versetzt. Nach dem Kühlen auf Raum-temperatur wird der feste Niederschlag abgesaugt, mit wenig Methanol und zuletzt gut mit warmem Wasser ausgewaschen. Es wird im Vakuum bei 70°C getrocknet und zur Reinigung aus Petroläther/Dioxan umkristallisiert. Man erhält die Verbindung der Formel 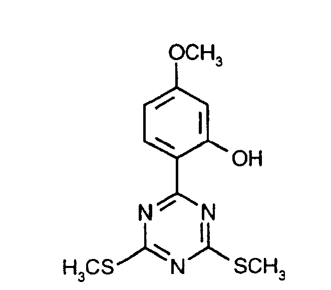 als weisses Pulver.
Beispiel 7: Zu einem Gemisch von 92,2 g Cyanurchlorid in 400 ml Toluol werden unter Rühren 250 g Eis eingetragen. Anschliessend tropft man innerhalb von ca. 1,5 h unter gutem Rühren eine Lösung von 61,9 g Natriumäthanthiolat in 250 ml Wasser ein, wobei sich die Innentemperatur allmählich bis auf etwa 30° C erhöht. Man rührt noch während 15 min bei ca. 48° C nach und lässt erkalten. Nach der Phasentrennung trennt man die wässerige Phase ab und wäscht die organische Phase dreimal mit je 500 ml Wasser. Man erhält nach dem Trocknen über Calciumchlorid 590,9 g toluolische Lösung, welche 11,5 Gew.-% 1-Aethylthio-3,5-dichlor-s-triazin und 3,48 Gew.-% 1,3-Bis-äthylthio-5-chlor-s-triazin enthält.
Beispiel 8: Zu 564,5 g der toluolischen Lösung aus Beispiel 7 trägt man unter gutem Rühren bei Raumtemperatur 95,8 g wasserfreies Aluminium(lll)chlorid ein, wobei sich unter schwacher Erwärmung ein rote Lösung bildet. Man erwärmt weiter bis auf 40° C und tropft innerhalb von 1,5 h eine Lösung von 79,2 g Resorcin in 165 ml Sulfolan ein. Die Innentemperatur erhöht sich dabei bis auf 57° C. Die Emulsion wird noch während 4 h bei 80° C nachgerührt. Die auf ca. 50° C abgekühlte Reaktionsmasse wird auf 1000 g Eis/250 ml Wasser ausgetragen. Toluol wird anschliessend azeotrop abdestilliert und die wässrige Produktsuspension abfiltriert. Man wäscht den Rückstand gut mit warmem und kaltem Wasser bis zur kongoneutralen Reaktion nach. Nach dem Trocknen im Vakuum bei 80° C erhält man 133,7 g eines hellgelben Pulvers, das die Verbindungen der Formeln 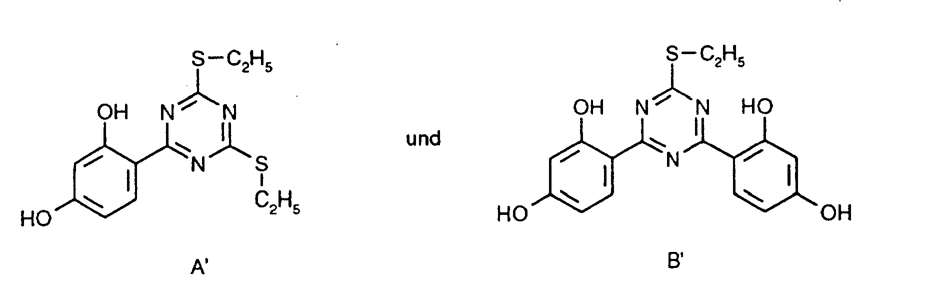 in einem Gewichtsverhältnis von A':B'=24:76 enthält.
Beispiel 9: Zu einem Gemisch von 20 g des Produktes aus Beispiel 8 in 100 ml Wasser werden 91,8 ml 2N Natronlauge eingetropft, wobei die Temperatur bei 20° C gehalten wird. Anschliessend tropft man innerhalb von 1,5 h 18,5 g Dimethylsulfat ein, wobei durch gleichzeitiges Zutropfen von 2N Natronlauge der pH-Wert des Reaktionsgemisches bei konstant 11 gehalten wird. Man rührt während 21 h bei Raumtemperatur nach und filtriert ab. Der Rückstand wird nochmals in Wasser angeschlämmt und mit verdünnter Salzsäure neutralisiert. Nach dem erneuten Filtrieren wird mit Wasser gut gewaschen und im Vakuum bei 70° C getrocknet. Man erhält ein beigefarbenes Pulver, das die Verbindungen der Formeln 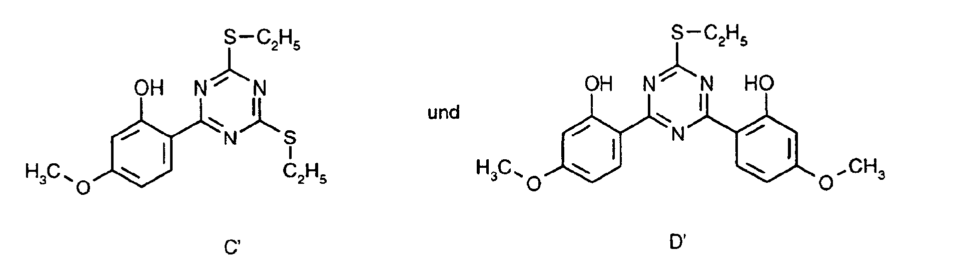 in einem Gewichtsverhältnis von C':D'=22:78 enthält.

### Färbebeispiele:

(i) Herstellung einer Stabilisatorformulierung:
   10 g des gemäss Beispiel 3 erhaltenen Stabilisatorgemisches werden mit 20 ml einer 10%igen wässrigen Lösung des Triethanolaminsalzes der Tristyrylphenyl-polyethylenglykolphosphorsäure und 25 g Quarzperlen (1mm Durchmesser) versetzt und 16 Stunden lang mit einem Schnellrührer gemahlen. Anschliessend wird das Mahlgut mit Hilfe eines feinmaschigen Netzes von den Glasperlen getrennt, unter Rühren mit 0,5 g Xanthangummi (z.B. Biopolymer^{®}AG) versetzt und mit Wasser auf einen Stabilisator-Gehalt von 10 Gew.-% eingestellt. Die Feinheit der Dispersion beträgt ca. 0,5-2µm.
(ii) Verwendung in der Ausziehfärberei:
   Ein 10g Muster eines Polyester-Trikots wird in einem HT-Färbeapparat (z.B. Turbomat^{®} der Firma Mathis, Niederhasli) mit einem Flottenverhältnis von 1:10 gefärbt. Die wässrige Färbe-flotte enthält 2 g/l Ammoniumsulfat, 0,5 g/l Färbereihilfsmittel (Univadin^{®} 3-flex), 0,5 Gew.-%, bezogen auf das Polyester-Trikot, der gemäss (i) hergestellten UV-Absorber-Formulierung und 0,89 Gew.-%, bezogen auf das Polyester-Trikot, einer Farbstoffmischung ent-haltend
   18,5 Gew.-% Farbstoff F₁ der Formel 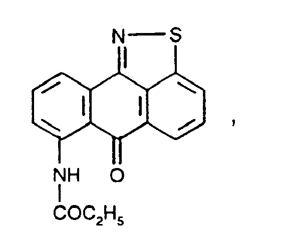
   18,5 Gew.-% Farbstoff F₂ der Formel 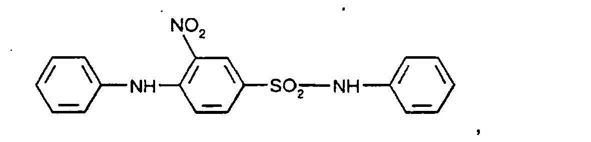
   17,4 Gew.-% Farbstoff F₃ der Formel 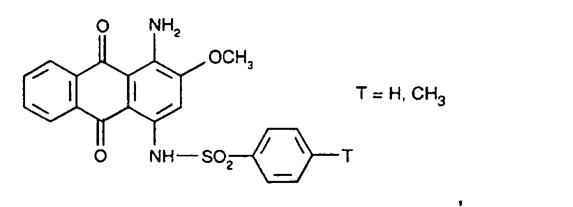
   11,6 Gew.-% Farbstoff F₄ der Formel 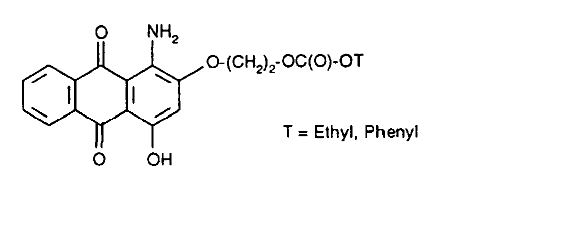
   14,3 Gew.-% Farbstoff F₅ der Formel 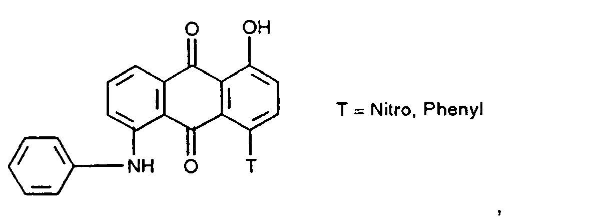
   10,2 Gew.-% Farbstoff F₆ der Formel 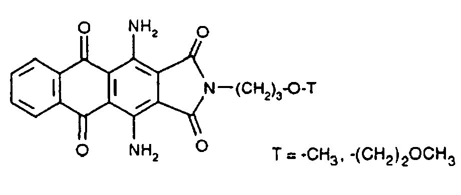 und
   9,5 Gew.-% Farbstoff F₇ der Formel

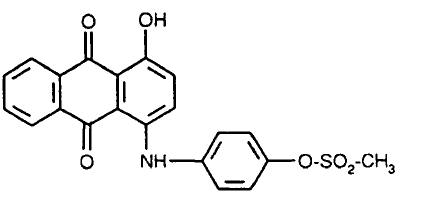

   Die Färbeflotte wird mit Essigsäure auf einen pH-Wert von 5 eingestellt, homogenisiert und mit dem Trikot in eine Druckfärbebombe gegeben. Man beginnt bei 70°C mit dem Färben, erhitzt dann innerhalb von 10 Minuten auf 100°C und innerhalb weiterer 20 Minuten auf 130°C. Nach einer Färbezeit von 30 Minuten bei dieser Temperatur kühlt man auf 50°C ab, spült das gefärbte Gewebe heiss und kalt und reinigt es reduktiv bei 70°C mit einer 3 ml/l 30%ige Natriumhydroxidlösung und 2 g/l Natriumdithionit enthaltenden Flotte. Nach dem Spülen und Trocknen ergibt sich ein grauviolett gefärbtes Polyester-Trikot, welches eine sehr gute Heisslichtechtheit aufweist.
(iii) Verfährt man wie unter (ii) beschrieben, färbt jedoch das Polyester Trikot mit einer Färbeflotte, die die gemäss (i) hergestellte Stabilisatorformulierung nicht enthält, wird eine Färbung mit einer deutlich geringeren Heisslichtechtheit erhalten.
(iv) Verfährt man wie unter (ii) beschrieben, färbt jedoch mit einer Färbeflotte, die 1,15 Gew.-%, bezogen auf das Polyester-Trikot, einer Farbstoffmischung enthaltend je 13 Gew.-% Farbstoff F₁ und F₂, 38,1 Gew.-% Farbstoff F₃, 25,4 Gew.-% Farbstoff F₄, 4,4 Gew.-% Farbstoff F₅, 3,2 Gew.-% Farbstoff F₆ und 2,9 Gew.-% Farbstoff F₇ erhält man ein weinrot gefärbtes Polyester-Trikot, welches eine sehr gute Heisslichtechtheit aufweist.
(v) Verfährt man wie unter (iv) beschrieben, färbt jedoch das Polyester Trikot mit einer Färbeflotte, die die gemäss (i) hergestellte Stabilisatorformulierung nicht enthält, wird eine Färbung mit einer deutlich geringeren Heisslichtechtheit erhalten.

Druckbeispiel: Zum Bedrucken von Polyester-Texturtrikot wird eine Druckpaste aus einer Stammverdickung, Farbstoffen und einer Stabilisatormischung hergestellt.

Die Stammverdickung hat folgende Zusammensetzung:

| | |
|---|---|
| 120 g | Stärkeether-Verdicker |
| 480 g | Natriumalginat-Verdicker |
| 5 g | Natriumdihydrogenphosphat |
| 5 g | Druckereihilfsmittel |
| 5 g | Natriumchlorat |
| 385 g | deionisiertes Wasser |
| 1000g | Stammverdickung |

Die Druckpaste weist folgende Zusammensetzung auf:

| | |
|---|---|
| 3,6 g | Farbstoff F₂ gemäss Färbebeispiel |
| 3,4 g | Farbstoffmischung enthaltend den Farbstoff F₃ gemäss Färbebeispiel und den Farbstoff der Formel |

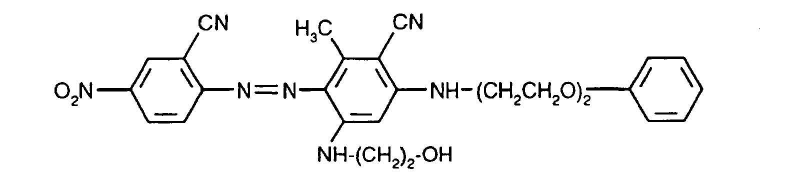

| | |
|---|---|
| 0,4 g | Farbstoff F₇ gemäss Färbebeispiel |
| 1,6 g | Farbstoff F₆ gemäss Färbebeispiel |
| 60 g | Stabilisatorgemisch gemäss Beispiel 3 als 20%ige Mahlung |
| 931 g | Stammverdickung. |

Mit dieser Druckpaste werden gereinigte Polyester-Trikotstücke auf einem Drucktisch bedruckt. Die Muster werden dann 10 Minuten bei 100°C getrocknet und anschliessend 8 Minuten mit überhitztem Dampf bei 180°C gedämpft. Man spült das bedruckte Gewebe heiss und kalt und reinigt es reduktiv bei 70°C mit einer 2 ml/l 30%ige Natriumhydroxidlösung und 3 g/l Natriumdithionit enthaltenden Flotte. Nach dem Spülen und Trocknen ergibt sich ein rotbeige bedrucktes Polyester-Trikot, welches eine sehr gute Heisslichtechtheit aufweist. Verfährt man wie oben beschrieben, verwendet jedoch eine Druckpaste, die die Stabilisatorformulierung nicht enthält, wird eine Färbung mit einer deutlich geringeren Heisslichtechtheit erhalten.

## Patentansprüche

1. Stabilisatorgemisch enthaltend im wesentlichen die Verbindungen der Formel 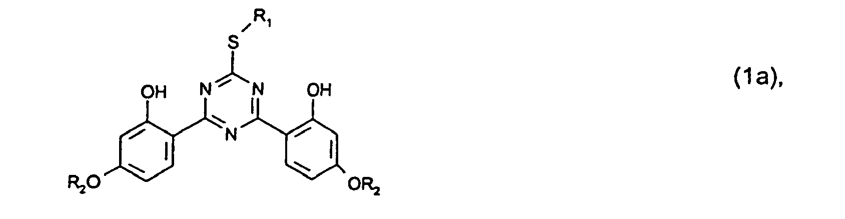 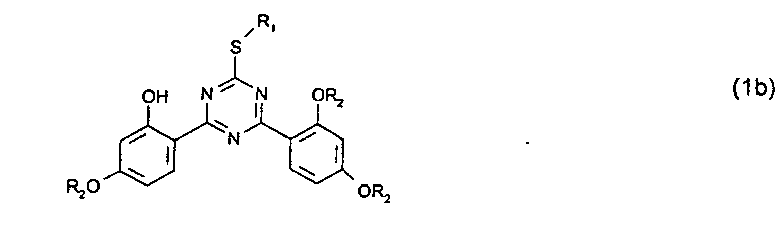 und 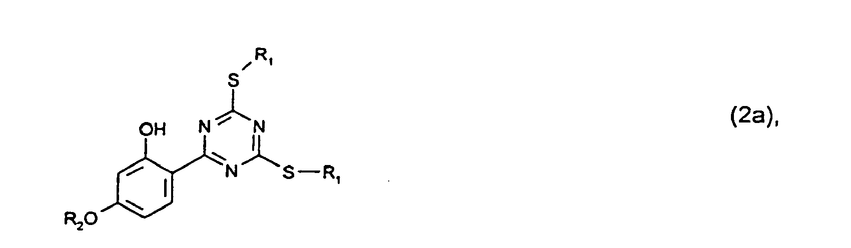 worin R₁ lineares oder verzweigtes C₁-C₁₈-Alkyl, C₃-C₈-Cycloalkyl, C₃-C₆-Alkenyl, gegebenenfalls substituiertes Aryl oder C₇-C₁₂-Aralkyl ist,
R₂ gegebenenfalls substituiertes lineares oder verzweigtes C₁-C₁₂-Alkyl, durch ein oder mehrere N-, S- oder O-Atome unterbrochenes lineares oder verzweigtes C₄-C₂₈-Alkyl, welches gegebenenfalls weitersubstituiert ist, oder einen Rest -CO-R₆ oder -SO₂R₆ bedeutet, und R₆ C₁-C₁₂-Alkyl, gegebenenfalls durch C₁-C₄-Alkyl substituiertes Phenyl oder C₇-C₁₂-Aralkyl ist.

2. Stabilisatorgemisch gemäss Anspruch 1, **dadurch gekennzeichnet, dass** R₁ C₁-C₆-Alkyl, unsubstituiertes oder durch 1-3 Methylgruppen substituiertes Cyclohexyl, Allyl, Isopropenyl, unsubstituiertes oder durch C₁-C₄-Alkyl, C₁-C₄-Alkoxy oder Halogen substituiertes Phenyl, Benzyl oder α-Methylbenzyl bedeutet.

3. Stabilisatorgemisch gemäss Anspruch 1 oder 2, **dadurch gekennzeichnet, dass** R₁ C₁-C₄-Alkyl, Cyclohexyl, Allyl, Phenyl oder Benzyl bedeutet.

4. Stabilisatorgemisch gemäss Anspruch 1, **dadurch gekennzeichnet, dass** R₁ C₁-C₄-Alkyl, besonders Methyl, bedeutet.

5. Stabilisatorgemisch gemäss einem der Ansprüche 1 bis 4, **dadurch gekennzeichnet, dass** R₂ für C₁-C₆-Alkyl oder für geradkettiges oder verzweigtes C₄-C₂₈-Alkyl, welches durch ein oder mehrere Gruppen -O- unterbrochen und durch Hydroxy oder einen Rest -OR₆, worin R₆ C₁-C₄-Alkyl, Phenyl, o-, m- oder p-Tolyl oder Benzyl ist, substituiert ist, oder für unsubstituiertes oder durch C₁-C₄-Alkyl substituiertes Benzoyl oder Phenylsulfonyl steht.

6. Stabilisatorgemisch gemäss einem der Ansprüche 1 bis 5, **dadurch gekennzeichnet, dass** R₂ C₁-C₄-Alkyl, ein Rest der Formel -(CH₂CHR₇-O)ₙ-H, worin R₇ Methyl oder insbesondere Wasserstoff und n eine ganze Zahl von 1 bis 9 bedeuten, oder Benzoyl bedeutet.

7. Stabilisatorgemisch gemäss einem der Ansprüche 1 bis 6, **dadurch gekennzeichnet, dass** R₂ C₁-C₄-Alkyl, ein Rest der Formel -(CH₂CHR₇-O)ₙ-H, worin R₇ Methyl oder insbesondere Wasserstoff und n eine ganze Zahl von 1 bis 9 bedeuten, oder Benzoyl ist.

8. Stabilisatorgemisch gemäss einem der Ansprüche 1 bis 7, **dadurch gekennzeichnet, dass** R₂ C₁-C₄-Alkyl bedeutet.

9. Stabilisatorgemisch gemäss Anspruch 1, enthaltend 50 bis 95 Gew.-% Verbindungen der Formeln (1a) und (1b) und 50 bis 5 Gew.-% der Verbindung der Formel (2a).

10. Stabilisatorgemisch gemäss Anspruch 1, enthaltend 50 bis 80 Gew.-% Verbindungen der Formeln (1a) und (1b) und 50 bis 20 Gew.-% der Verbindung der Formel (2a), worin R₁ C₁-C₄-Alkyl, Cyclohexyl, Allyl, Phenyl oder Benzyl und R₂ C₁-C₄-Alkyl, ein Rest der Formel - (CH₂CHR₄-O)ₙ-H, worin R₄ Methyl oder insbesondere Wasserstoff und n eine ganze Zahl von 1 bis 9 bedeuten, oder Benzoyl sind.

11. Verfahren zur Herstellung eines Stabilisatorgemisches gemäss einem der Ansprüche 1 bis 10, **dadurch gekennzeichnet, dass** man .
(i) ein Cyanurhalogenid in wässrig-organischer Lösung mit einem Überschuss einer Verbindung der Formel
R₁ -S-X (3)
worin X Wasserstoff oder ein Kation ist und R₁ die im Anspruch 1 angegebene Bedeutung hat, gegebenenfalls in Gegenwart eines Halogenwasserstoffakzeptors, welcher in Fall von X = Wasserstoff anwesend sein muss, kondensiert,
(ii) das gemäss (i) erhältliche die Verbindungen der Formeln 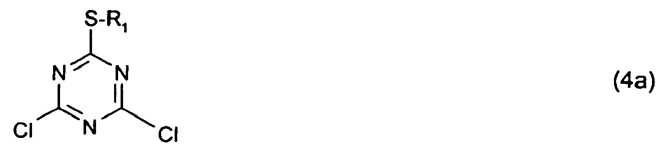 und 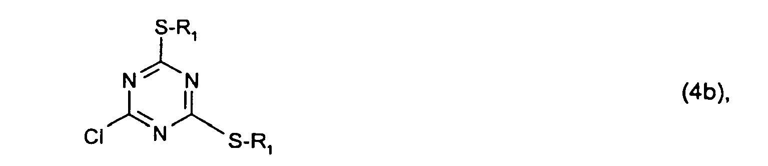 enthaltende Gemisch in Gegenwart eine Lewis-Säure mit Resorcin umsetzt, und
(iii) das gemäss (ii) erhältliche die Verbindungen der Formeln 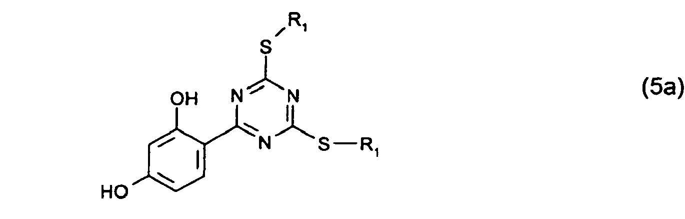 und 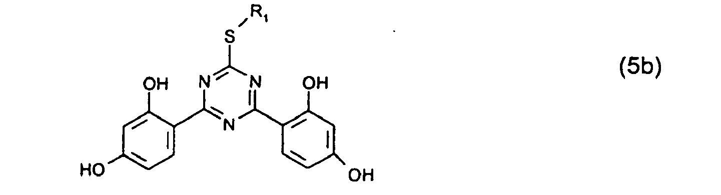
enthaltende Gemisch mit einem den Rest R₂, enthaltenden Alkylierungs- oder Acylierungsmittel in ein erfindungsgemässes Stabilisatorgemisch überführt.

12. Verfahren zum Stabilisieren von organischem Material gegen Schädigung durch Licht, Sauerstoff und/oder Hitze, **dadurch gekennzeichnet, dass** man diesem ein Stabilisatorgemisch enthaltend je mindestens eine Verbindung der im Anspruch 1 angegebenen Formeln (1a), (1 b) und (2a) zusetzt.

13. Verwendung von Stabilisatorgemischen enthaltend mindestens je eine Verbindung der im Anspruch 1 angegebenen Formeln (1a), (1b) und (2a) zum Stabilisieren von organischem Material gegen Schädigung durch Licht, Sauerstoff und/oder Hitze.

14. Verfahren zur photochemischen und thermischen Stabilisierung von ungefärbten, gefärbten oder bedruckten Textilfasermaterialien, **dadurch gekennzeichnet, dass** man die Textilfasermaterialien mit einem Stabilisatorgemisch enthaltend mindestens je eine Verbindung der im Anspruch 1 angegebenen Formeln (1a), (1b) und (2a) behandelt.

15. Verfahren gemäss Anspruch 14 zur photochemischen und thermischen Stabilisierung von ungefärbten, gefärbten oder bedruckten Polyester-Fasermaterialien.

16. Verfahren zur Erhöhung des Sonnenschutzfaktors (UPF) von ungefärbten, gefärbten oder bedruckten Textilfasermaterialien, **dadurch gekennzeichnet, dass** man die Textilfasermaterialien mit einem Stabilisatorgemisch enthaltend mindestens je eine Verbindung der im Anspruch 1 angegebenen Formeln (1a), (1b) und (2a) behandelt.

17. Verwendung von Stabilisatorgemischen enthaltend mindestens je eine Verbindung der im Anspruch 1 angegebenen Formeln (1a), (1b) und (2a) zur photochemischen und thermischen Stabilisierung von ungefärbten, gefärbten oder bedruckten Textilfasermaterialien.

18. Verbindung der Formel

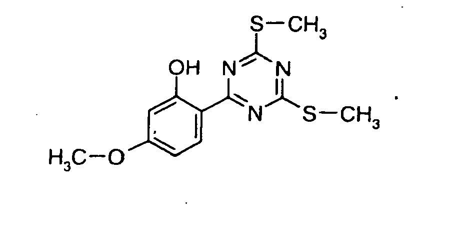

## Claims

1. A stabilizer mixture comprising essentially the compounds of the formula 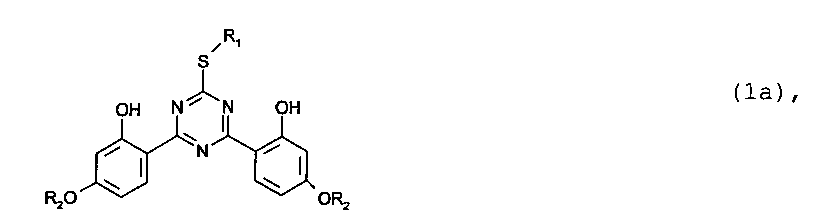 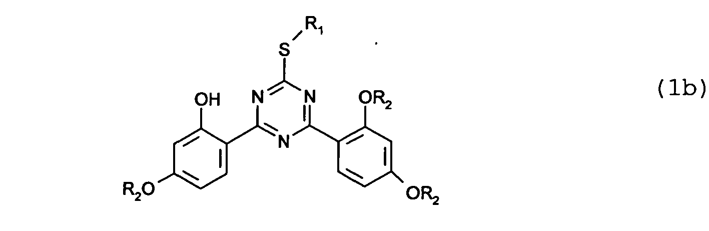 and 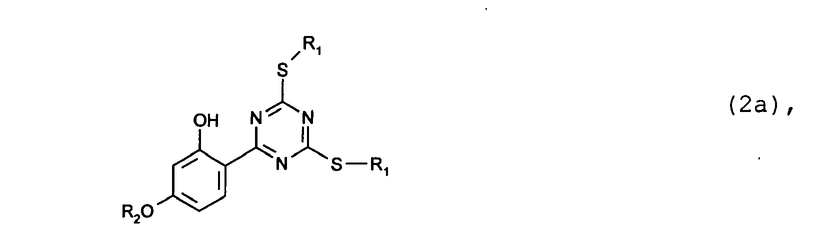 in which R₁ is linear or branched C₁-C₁₈alkyl,
C₃-C₈cycloalkyl, C₃-C₆alkenyl, unsubstituted or substituted aryl or C₇-C₁₂aralkyl, R₂ is unsubstituted or substituted, linear or branched C₁-C₁₂alkyl, or linear or branched C₄-C₂₈alkyl, which is interrupted by one or more N, S or O atoms and which may be substituted further or is a radical -CO-R₆ or -SO₂-R₆, and R₆ is C₁-C₁₂alkyl, unsubstituted or C₁-C₄alkyl-substituted phenyl or C₇-C₁₂aralkyl.

2. A stabilizer mixture according to claim 1, wherein R₁ is C₁-C₆alkyl, cyclohexyl which is unsubstituted or substituted by 1-3 methyl groups, allyl, isopropenyl, unsubstituted or C₁-C₄alkyl-, C₁-C₄alkoxy- or halo-substituted phenyl, benzyl or □-methylbenzyl.

3. A stabilizer mixture according to claim 1 or 2, wherein R₁ is C₁-C₄alkyl, cyclohexyl, allyl, phenyl or benzyl.

4. A stabilizer mixture according to claim 1, wherein R₁ is C₁-C₄alkyl, especially methyl.

5. A stabilizer mixture according to any one of claims 1 to 4, wherein R₂ is C₁-C₆alkyl or straight-chain or branched C₄-C₂₈alkyl which is interrupted by one or more groups -O- and is substituted by hydroxyl or by a radical -OR₆, in which R₆ is C₁-C₄alkyl, phenyl, o-, m- or p-tolyl or benzyl, or is unsubstituted or C₁-C₄alkyl-substituted benzoyl or phenylsulfonyl.

6. A stabilizer mixture according to any one of claims 1 to 5, wherein R₂ is C₁-C₄alkyl, a radical of the formula -(CH₂CHR₇-O)ₙ-H, in which R₇ is methyl or, in particular, hydrogen and n is an integer from 1 to 9, or is benzoyl.

7. A stabilizer mixture according to any one of claims 1 to 6, wherein R₂ is C₁-C₄alkyl, a radical of the formula -(CH₂CHR₇-O)ₙ-H, in which R₇ is methyl or, in particular, hydrogen and n is an integer from 1 to 9, or is benzoyl.

8. A stabilizer mixture according to any one of claims 1 to 7, wherein R₂ is C₁-C₄alkyl.

9. A stabilizer mixture according to claim 1, comprising from 50 to 95% by weight of compounds of the formulae (1a) and (1b) and from 50 to 5% by weight of the compound of the formula (2a).

10. A stabilizer mixture according to claim 1, comprising from 50 to 80% by weight of compounds of the formulae (1a) and (1b) and from 50 to 20% by weight of the compound of the formula (2a), in which R₁ is C₁-C₄alkyl, cyclohexyl, allyl, phenyl or benzyl and R₂ is C₁-C₄alkyl, a radical of the formula -(CH₂CHR₄-O)ₙ-H, in which R₄ is methyl or, in particular, hydrogen and n is an integer from 1 to 9, or is benzoyl.

11. A process for preparing a stabilizer mixture according to any one of claims 1 to 10, which comprises
(i) condensing a cyanuric halide in aqueous-organic solution with an excess of a compound of the formula
R₁ -S-X (3)
in which X is hydrogen or a cation and R₁ is as defined in claim 1, in the presence or absence of a hydrogen halide acceptor, which must be present if X = hydrogen,
(ii) reacting the mixture obtainable in accordance with (i), comprising the compounds of the formulae 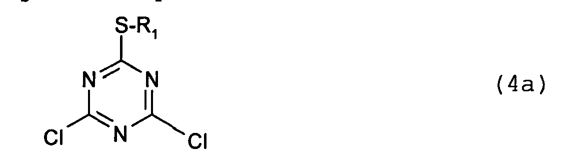 and 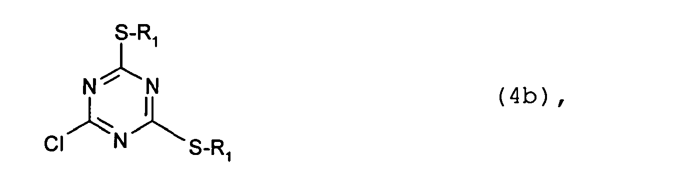 with resorcinol in the presence of a Lewis acid, and
(iii) converting the mixture obtainable in accordance with (ii), comprising the compounds of the formulae 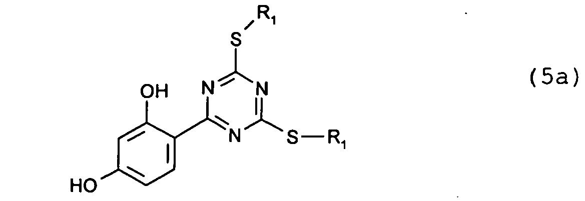 and 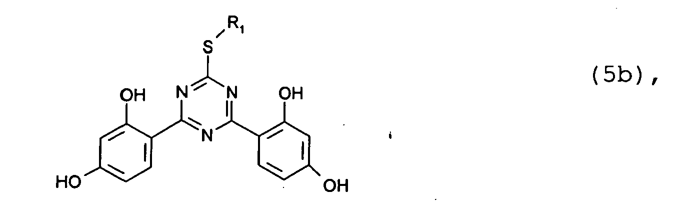
with an acylating agent or alkylating agent comprising the radical R₂, into a novel stabilizer mixture.

12. A process for stabilizing organic material against damage by light, oxygen and/or heat, which comprises adding thereto a stabilizer mixture comprising in each case at least one compound of the formulae (1a), (1b) and (2a) indicated in claim 1.

13. The use of stabilizer mixtures comprising at least one compound each of the formulae (1a), (1b) and (2a) indicated in claim 1 for stabilizing organic material against damage by light, oxygen and/or heat.

14. A process for the photochemical and thermal stabilization of undyed, dyed or printed textile fibre materials, which comprises treating the textile fibre materials with a stabilizer mixture comprising at least one compound each of the formulae (1a), (1b) and (2a) indicated in claim 1.

15. A process according to claim 14 for the photochemical and thermal stabilization of undyed, dyed or printed polyester fibre materials.

16. A process for increasing the sun protection factor (UPF) of undyed, dyed or printed textile fibre materials, which comprises treating the textile fibre materials with a stabilizer mixture comprising at least one compound each of the formulae (1a), (1b) and (2a) indicated in claim 1.

17. The use of a stabilizer mixture comprising at least one compound each of the formulae (1a), (1b) and (2a) indicated in claim 1 for the photochemical and thermal stabilization of undyed, dyed or printed textile fibre materials.

18. A compound of the formula

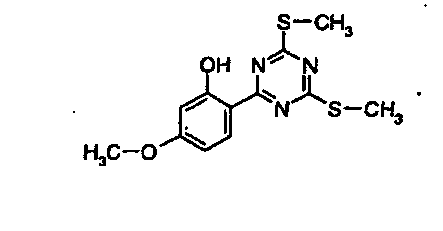

## Revendications

1. Mélange de stabilisateurs contenant essentiellement les composés répondant aux formules : 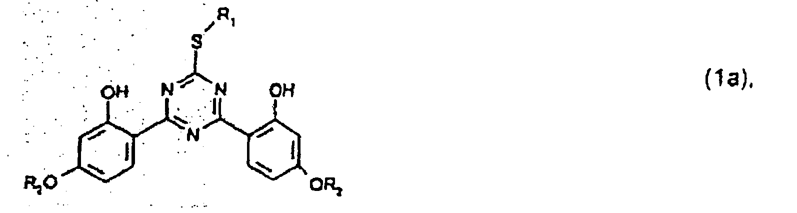 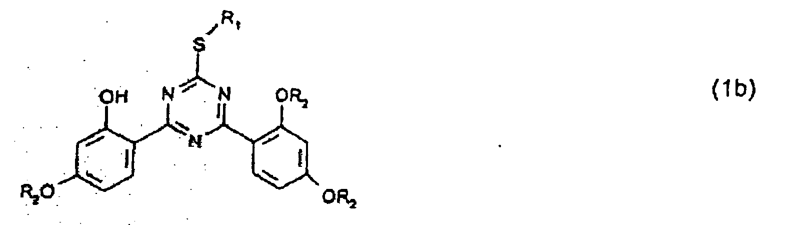 et 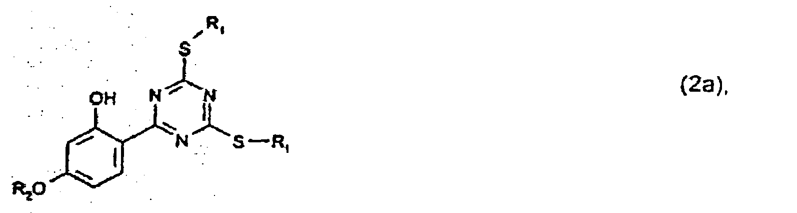 dans lesquelles R₁ représente un groupe alkyle en C₁-C₁₈, cycloalkyle en C₃-C₈, alcényle en C₃-C₆, linéaire ou ramifié, un groupe aryle ou un groupe aralkyle en C₇-C₁₂ éventuellement substitués,
R₂ représente un groupe alkyle en C₁-C₁₂ linéaire ou ramifié éventuellement substitué, un groupe alkyle en C₄-C₂₈ linéaire ou ramifié interrompu par un ou plusieurs atomes de N, S ou O et éventuellement encore substitué, ou représente un radical -CO-R₆ ou -SO₂-R₆, et R₆ représente un groupe alkyle en C₁-C₁₂, un groupe phényle ou aralkyle en C₇-C₁₂ éventuellement substitué par un groupe alkyle en C₁-C₄.

2. Mélange de stabilisateurs selon la revendication 1, **caractérisé en ce que** R₁ représente un groupe alkyle en C₁-C₆, un groupe cyclohexyle, allyle, isopropényle non substitué ou substitué par 1 à 3 groupes méthyle, un groupe phényle, benzyle ou α-méthylbenzyle non substitué ou substitué par un groupe alkyle en C₁-C₄, alcoxy en C₁-C₄ ou un halogène.

3. Mélange de stabilisateurs selon la revendication 1 ou 2, **caractérisé en ce que** R₁ représente un groupe alkyle en C₁-C₄, cyclohexyle, allyle, phényle ou benzyle.

4. Mélange de stabilisateurs selon la revendication 1, **caractérisé en ce que** R₁ représente un groupe alkyle en C₁-C₄, notamment méthyle.

5. Mélange de stabilisateurs selon l'une des revendications 1 à 4, **caractérisé en ce que** R₂ représente un groupe alkyle en C₁-C₆ ou un groupe alkyle en C₄-C₂₈ à chaîne droite ou ramifiée interrompu par un ou plusieurs groupes -O- et substitué par un groupe hydroxy ou un radical -OR₆, dans lequel R₆ représente un groupe alkyle en C₁-C₄, phényle, o-, m- ou p-tolyle ou benzyle, ou bien R₂ représente un groupe benzoyle ou phénylsulfonyle non substitué ou substitué par un groupe alkyle en C₁-C₄.

6. Mélange de stabilisateurs selon l'une des revendications 1 à 5, **caractérisé en ce que** R₂ représente un groupe alkyle en C₁-C₄, un radical répondant à la formule -(CH₂CHR₇-O)ₙ-H, dans laquelle R₇ représente un groupe méthyle ou notamment l'hydrogène et n est un nombre entier valant de 1 à 9, ou bien R₂ représente un groupe benzoyle.

7. Mélange de stabilisateurs selon l'une des revendications 1 à 6, **caractérisé en ce que** R₂ représente un groupe alkyle en C₁-C₄, un radical répondant à la formule -(CH₂CHR₇-O)ₙ-H, dans laquelle R₇ représente un groupe méthyle ou notamment l'hydrogène et n est un nombre entier valant de 1 à 9, ou bien R₂ représente un groupe benzoyle.

8. Mélange de stabilisateurs selon l'une des revendications 1 à 7, **caractérisé en ce que** R₂ représente un groupe alkyle en C₁-C₄.

9. Mélange de stabilisateurs selon la revendication 1, contenant 50 à 95 % en poids de composés répondant aux formules (1a) et (1b) et 50 à 5 % en poids de composé répondant à la formule (2a).

10. Mélange de stabilisateurs selon la revendication 1, contenant 50 à 80 % en poids de composés répondant aux formules (1a) et (1b) et 50 à 20 % en poids de composé répondant à la formule (2a), dans lesquelles R₁ représente un groupe alkyle en C₁-C₄, cyclohexyle, allyle, phényle ou benzyle et R₂ représente un groupe alkyle en C₁-C₄, un radical répondant à la formule -(CH₂CHR₄-O)ₙ-H, dans laquelle R₄ représente un groupe méthyle ou notamment l'hydrogène et n est un nombre entier valant de 1 à 9, ou bien R₂ représente un groupe benzoyle.

11. Procédé de préparation d'un mélange de stabilisateurs selon l'une des revendications 1 à 10, **caractérisé en ce que** :
(i) on condense un halogénure cyanurique en solution organique aqueuse avec un excédent d'un composé répondant à la formule :
R₁-S-X (3)
dans laquelle X représente l'hydrogène ou un cation et R₁ a la signification indiquée dans la revendication 1, en la présence éventuelle d'un accepteur d'hydracide halogéné qui doit être présent lorsque X = hydrogène,
(ii) on fait réagir avec de la résorcine le mélange obtenu selon (i) et contenant les composés répondant aux formules : 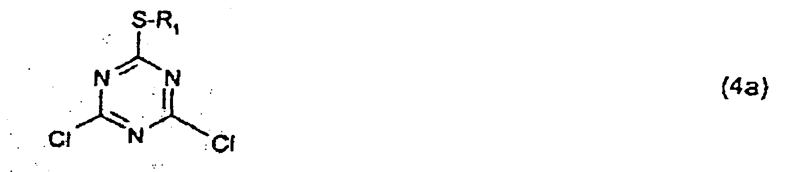 et 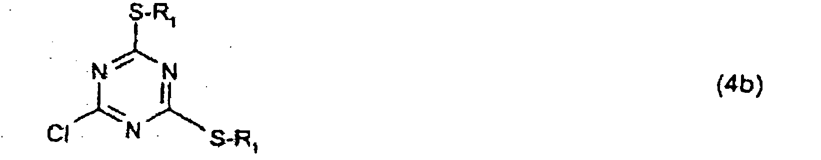 en présence d'un acide de Lewis, et
(iii) on transfère le mélange obtenu selon (ii) et contenant les composés répondant aux formules : 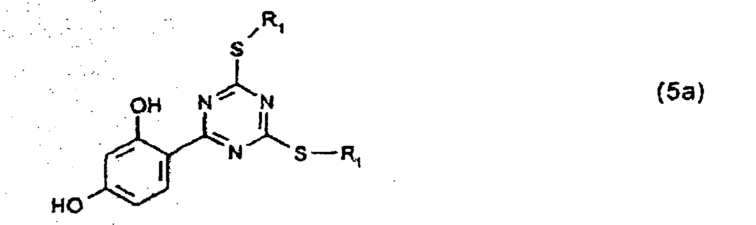 et 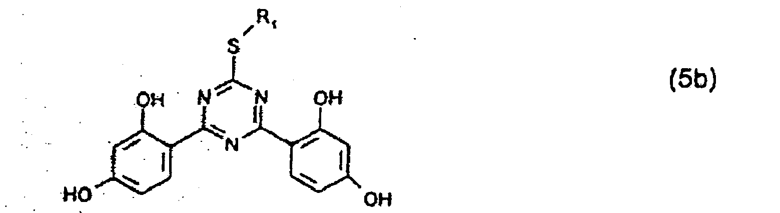
avec un agent d'alkylation ou d'acylation contenant le radical R₂ dans un mélange de stabilisateurs selon l'invention.

12. Procédé de stabilisation de matériau organique contre les dommages causés par la lumière, l'oxygène et/ou la chaleur, **caractérisé en ce qu'**on ajoute à ce matériau un mélange de stabilisateurs contenant respectivement au moins un composé répondant aux formules (1a), (1b) et (2a) indiquées dans la revendication 1.

13. Utilisation de mélange de stabilisateurs contenant respectivement au moins un composé répondant aux formules (1a), (1b) et (2a) indiquées dans la revendication 1 pour stabiliser un matériau organique contre les dommages causés par la lumière, l'oxygène et/ou la chaleur.

14. Procédé de stabilisation photochimique et thermique de matériaux en fibres textiles teints, non teints ou imprimés, **caractérisé en ce qu'**on traite les matériaux en fibres textiles avec un mélange de stabilisateurs contenant respectivement au moins un composé répondant aux formules (1a), (1b) et (2a) indiquées dans la revendication 1.

15. Procédé selon la revendication 14 en vue de la stabilisation photochimique et thermique de matériaux en fibres de polyester teints, non teints ou imprimés.

16. Procédé d'élévation du facteur de protection solaire (UPF) de matériaux en fibres textiles teints, non teints ou imprimés, **caractérisé en ce qu'**on traite les matériaux en fibres textiles avec un mélange de stabilisateurs contenant respectivement au moins un composé répondant aux formules (1a), (1b) et (2a) indiquées dans la revendication 1.

17. Utilisation de mélanges de stabilisateurs contenant respectivement au moins un composé répondant aux formules (1a), (1b) et (2a) indiquées dans la revendication 1 pour la stabilisation photochimique et thermique de matériaux en fibres textiles teints, non teints ou imprimés.

18. Composé répondant à la formule :
